# EUROPEAN PATENT APPLICATION

(11) **EP 4 674 870 A1**
(43) Date of publication of application: **07.01.2026**
(21) Application number: 25751307.7
(22) Date of filing: 23.01.2025
(51) Int. Cl.: C07K 16/28, A61K 39/395, C12N 15/11, A61P 37/06, A61P 1/00

(54) **ANTI-TL1A ANTIBODY, PREPARATION METHOD THEREFOR AND USE THEREOF**

(30) Priority: 05.02.2024 CN 202410160677
(71) Applicant: Sunshine Guojian Pharmaceutical (Shanghai) Co., Ltd., Shanghai 201203 (CN)
(72) Inventor: DENG, Lan, Shanghai 201203 (CN); HUANG, Haomin, Shanghai 201203 (CN); LOU, Jing, Shanghai 201203 (CN); WANG, Lihua, Shanghai 201203 (CN); YU, Zhilun, Shanghai 201203 (CN); MENG, Yun, Shanghai 201203 (CN)
(74) Representative: dompatent
(86) International application number: PCT/CN2025/074378
(87) International publication number: WO 2025/167705

(57) **Abstract**

Disclosed in the present invention are an anti-TL1A antibody, a preparation method therefor and the use thereof. The antibody comprises a heavy chain variable region and a light chain variable region, and has one or more of the following technical features: the heavy chain variable region comprises HCDR1 having an amino acid sequence as shown in SEQ ID NO: 10 or 18; the heavy chain variable region comprises HCDR2 having an amino acid sequence as shown in SEQ ID NO: 11, 19, 39, 40 or 41; the heavy chain variable region comprises HCDR3 having an amino acid sequence as shown in SEQ ID NO: 12, 20 or 42; the light chain variable region comprises LCDR1 having an amino acid sequence as shown in SEQ ID NO: 13 or 21; the light chain variable region comprises LCDR2 having an amino acid sequence as shown in SEQ ID NO: 14 or 22; and the light chain variable region comprises LCDR3 having an amino acid sequence as shown in SEQ ID NO: 15 or 23. The antibody of the present invention can inhibit TL1A-induced IFN-y and TNF-α secretion by PBMCs, and can inhibit TL1A-induced apoptosis, thus prompting that the antibody or a composition thereof can be used for treating diseases such as inflammatory bowel disease, and gastrointestinal diseases associated with cystic fibrosis.

## Description

### TECHNICAL FIELD

The present invention relates to the field of antibody drugs, and in particular to an anti-TL1A antibody and a preparation method and use thereof.

### BACKGROUND ART

Inflammatory bowel disease (IBD) is a specific chronic inflammatory intestinal disease, including Crohn's disease (CD) and ulcerative colitis (UC). The etiology and pathogenesis of inflammatory bowel disease remain unclear, and it is generally believed to result from the interaction of multiple factors, such as abnormal intestinal mucosal immune regulation, persistent intestinal infection, defects in the intestinal mucosal barrier, genetic and environmental factors. Both innate and adaptive immune responses of the intestinal mucosa are involved in the pathological formation process of IBD, and abnormal immune responses in the intestinal mucosa are an important cause of mucosal tissue damage.

TL1A (also known as tumor necrosis factor superfamily member 15 [TNFSF15]) is a member of the TNF ligand superfamily and, like other members, is a homotrimer. TL1A is a type II transmembrane protein that exists in both membrane-bound and soluble forms, and its receptors include DR3 and soluble decoy receptor 3 (DcR3). DR3, also known as APO3, LARD, or WSL1, is a member of the tumor necrosis factor receptor superfamily (TNFRSF). TL1A is a ligand of DR3. Upon binding, it can induce nuclear factor-kappa B (NF-κB) activation, promote the transcription of inflammatory factors, and activate apoptotic proteases, thereby exerting pro-inflammatory and pro-apoptotic effects. DcR3 competitively binds to TL1A, thereby attenuating the generation of T-cell co-stimulatory signals and exerting anti-apoptotic and inhibitory effects on the secretion of pro-inflammatory factors.

Recent studies have found that TL1A can promote intestinal mucosal inflammatory responses and fibrosis by affecting the activation and proliferation of T cells in the intestinal epithelial lamina propria, promoting the polarization and effector functions of Th1 cells, and upregulating the expression of IFN-γ. Simultaneously, TL1A can also act on Th17 cells to increase their secretion of IL-17, which disrupts intestinal mucosal immunity and induces an inflammatory response. Chronic inflammatory responses are the primary factor in the occurrence of intestinal fibrosis, and inflammatory responses can further activate intestinal fibroblasts, resulting in the production of large amounts of ECM, thereby contributing to the formation of intestinal fibrosis. Therefore, TL1A may promote the occurrence of chronic experimental colitis-associated intestinal fibrosis by regulating IL-17 and IFN-γ. Anti-TL1A antibodies can effectively inhibit TL1A-induced secretion of IFN-γ and TNF-α by human peripheral blood mononuclear cells (PBMC), and can inhibit TL1A-induced apoptosis, thus being useful for treating inflammatory bowel disease and related disorders.

### SUMMARY OF THE INVENTION

In view of the drawbacks of the prior art described above, an object of the present invention is to provide an anti-TL1A antibody, a preparation method therefor, and uses thereof, so as to solve the problems in the prior art.

To achieve the above object and other related objects, the present invention provides an anti-TL1A antibody or an antigen-binding fragment thereof, wherein the antibody or antigen-binding fragment thereof comprises a heavy chain variable region and a light chain variable region, and the anti-TL1A antibody or antigen-binding fragment thereof has one or more of the following technical features:
<1> the heavy chain variable region comprises an HCDR1 having an amino acid sequence as shown in SEQ ID NO: 10 or 18;
<2> the heavy chain variable region comprises an HCDR2 having an amino acid sequence as shown in SEQ ID NO: 11, 19, 39, 40, or 41;
<3> the heavy chain variable region comprises an HCDR3 having an amino acid sequence as shown in SEQ ID NO: 12, 20, or 42;
<4> the light chain variable region comprises an LCDR1 having an amino acid sequence as shown in SEQ ID NO: 13 or 21;
<5> the light chain variable region comprises an LCDR2 having an amino acid sequence as shown in SEQ ID NO: 14 or 22;
<6> the light chain variable region comprises an LCDR3 having an amino acid sequence as shown in SEQ ID NO: 15 or 23.

The present invention also provides a recombinant protein, wherein the recombinant protein comprises: (i) the anti-TL1A antibody or antigen-binding fragment thereof; and (ii) optionally a tag sequence that facilitates expression and/or purification.

The present invention also provides an isolated polynucleotide which encodes the heavy chain variable region and/or light chain variable region or full-length amino acid sequence of the anti-TL1A antibody or antigen-binding fragment thereof, or encodes the recombinant protein.

The present invention also provides a nucleic acid construct comprising the isolated polynucleotide.

The present invention also provides a cell comprising the construct or having the exogenous polynucleotide integrated into its genome.

The present invention also provides a method for preparing the anti-TL1A antibody or antigen-binding fragment thereof, comprising the step of culturing the cell under conditions suitable for expressing the TL1A antibody or antigen-binding fragment thereof, thereby expressing the TL1A antibody or antigen-binding fragment thereof; further, the method also comprises the step of purifying and/or isolating the TL1A antibody or antigen-binding fragment thereof.

The present invention also provides a use of the anti-TL1A antibody or antigen-binding fragment thereof or the recombinant protein in the preparation of a medicament for treating a disease or in the preparation of a medicament for diagnosing a disease.

In the present invention, the disease is selected from one or more of inflammatory bowel disease, gastrointestinal disease associated with cystic fibrosis, colitis, irritable bowel syndrome, eosinophilic esophagitis, atopic dermatitis, eczema, scleroderma, arthritis or rheumatoid arthritis. Wherein the inflammatory bowel disease is Crohn's disease or ulcerative colitis.

The present invention also provides a pharmaceutical composition comprising the anti-TL1A antibody or antigen-binding fragment thereof or the recombinant protein. The present invention also provides a method for treating a disease, the method comprising administering to a subject in need thereof the anti-TL1A antibody or antigen-binding fragment thereof, the recombinant protein, or the pharmaceutical composition of the present invention.

As described above, the anti-TL1A antibody or antigen-binding fragment thereof of the present invention has the following beneficial effects: it can effectively inhibit the secretion of IFN-γ and TNF-α by TL1A-induced human peripheral blood mononuclear cells (PBMC), and can inhibit TL1A-induced apoptosis, indicating that the antibody or composition thereof can be used for treating inflammatory bowel disease, gastrointestinal disease associated with cystic fibrosis, Crohn's disease, colitis, ulcerative colitis, irritable bowel syndrome, eosinophilic esophagitis, atopic dermatitis, eczema, scleroderma, arthritis, or rheumatoid arthritis.

It should be understood that, within the scope of the present invention, the various technical features of the present invention described above and the various technical features specifically described below (such as in the examples) can be combined with each other to form new or preferred technical solutions, which will not be repeated here one by one due to space limitation.

### BRIEF DESCRIPTION OF DRAWINGS

Figures 1A to 1C show the binding ability results of the test murine antibodies to the target antigen human TL1A-His in Example 1.
Figures 2A to 2C show the blocking activity determination results of the test murine antibodies against the binding of TL1A protein to the stable transfection cell line 293FT-DR3 in Example 1.
Figure 3A shows the ELISA detection results of the binding affinity of candidate antibodies obtained after physicochemical property optimization of humanized antibodies 72851 and 31122, as well as humanized antibody 31122, to the antigen TL1A-his in Example 4.
Figure 3B shows the capillary isoelectric focusing detection results of candidate antibodies obtained after physicochemical property optimization of humanized antibodies 72851 and 31122, as well as humanized antibody 31122, in Example 4.
Figure 4 shows the results of the experiment on the blocking of TL1A binding to DR3 by anti-TL1A antibodies in Example 5.
Figure 5 shows the experimental results of the inhibition of the NF-κB signaling pathway in TL1A TF-1 cell lines by anti-TL1A antibodies in Example 6.
Figure 6 shows the results of inhibition of TL1A-induced apoptosis in TF-1 cells by anti-TL1A antibodies in Example 7.
Figure 7 shows the results of inhibition of TL1A-stimulated IFNγ secretion by anti-TL1A antibodies in Example 8.
Figure 8 shows the results of inhibition of TL1A-stimulated TNFα secretion by anti-TL1A antibodies in Example 9.
Figure 9A shows the ELISA detection results of the binding affinity of humanized antibody 72581 to TL1A proteins from human, cynomolgus monkey, rat, and mouse in Example 10.
Figures 9B to 9E show the efficacy results of anti-TL1A antibodies in a DSS-induced chronic inflammatory bowel disease model in rats in Example 10.
Figures 10A and 10B show the efficacy results of anti-TL1A antibodies in a TNBS-induced acute inflammatory bowel disease model in rats in Example 11.

### DETAILED DESCRIPTION OF THE PREFERRED EMBODIMENTS

The present invention provides an anti-TL1A antibody or antigen-binding fragment thereof, wherein the antibody or antigen-binding fragment thereof comprises a heavy chain variable region and a light chain variable region, and the anti-TL1A antibody or antigen-binding fragment thereof has one or more of the following technical features:
<1> the heavy chain variable region comprises an HCDR1 having an amino acid sequence as shown in SEQ ID NO: 10 or 18;
<2> the heavy chain variable region comprises an HCDR2 having an amino acid sequence as shown in SEQ ID NO: 11, 19, 39, 40, or 41;
<3> the heavy chain variable region comprises an HCDR3 having an amino acid sequence as shown in SEQ ID NO: 12, 20, or 42;
<4> the light chain variable region comprises an LCDR1 having an amino acid sequence as shown in SEQ ID NO: 13 or 21;
<5> the light chain variable region comprises an LCDR2 having an amino acid sequence as shown in SEQ ID NO: 14 or 22;
<6> the light chain variable region comprises an LCDR3 having an amino acid sequence as shown in SEQ ID NO: 15 or 23. In some embodiments of the present invention, the anti-TL1A antibody or antigen-binding fragment thereof comprises:
   <1> a heavy chain variable region comprising an HCDR1 having an amino acid sequence as shown in SEQ ID NO: 10;
   <2> a heavy chain variable region comprising an HCDR2 having an amino acid sequence as shown in SEQ ID NO: 11;
   <3> a heavy chain variable region comprising an HCDR3 having an amino acid sequence as shown in SEQ ID NO: 12;
   <4> a light chain variable region comprising an LCDR1 having an amino acid sequence as shown in SEQ ID NO: 13;
   <5> a light chain variable region comprising an LCDR2 having an amino acid sequence as shown in SEQ ID NO: 14;
   <6> a light chain variable region comprising an LCDR3 having an amino acid sequence as shown in SEQ ID NO: 15.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 19;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 39;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 40;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 41;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 39;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 40;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

In some embodiments of the present invention, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 41;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

Complementarity determining region (CDR) generally refers to a region in an antibody that structurally complements an antigenic determinant. Variability in antibodies is generally not uniformly distributed throughout the variable regions of the antibody. Both the heavy chain variable region and the light chain variable region of a monoclonal antibody typically comprise three hypervariable regions (HVRs), which structurally complement antigenic determinants. Therefore, hypervariable regions are also referred to as complementarity determining regions (CDRs). That is, the heavy chain variable region generally comprises three complementarity determining regions, namely HCDR1, HCDR2, and HCDR3, and the light chain variable region generally comprises three complementarity determining regions, namely LCDR1, LCDR2, and LCDR3.

In some embodiments of the present invention, the heavy chain variable region and the light chain variable region may further comprise framework regions. The framework regions may be located between the complementarity determining regions or at both ends of the complementarity determining regions. In some specific embodiments of the present invention, the sequence of the framework region is derived from the variable region of a human monoclonal antibody, or is a framework region sequence obtained by substitution, deletion, or addition of one or more (specifically 1 to 50, 1 to 30, 1 to 20, 1 to 10, 1 to 5, or 1 to 3) amino acids to the framework region sequence of a murine monoclonal antibody variable region. This framework region sequence may share 80%, 85%, 90%, 93%, 95%, 97%, or more than 99% homology with the framework region sequence of a human monoclonal antibody variable region.

In some embodiments of the present invention, the amino acid sequence of the heavy chain variable region of the anti-TL1A antibody or antigen-binding fragment thereof is as shown in any one of SEQ ID NO: 3, SEQ ID NO: 16, SEQ ID NO: 7, SEQ ID NO: 24, or SEQ ID NO: 26-31.

In some embodiments of the present invention, the amino acid sequence of the light chain variable region of the anti-TL1A antibody or antigen-binding fragment thereof is as shown in SEQ ID NO: 5, SEQ ID NO: 17, SEQ ID NO: 9, or SEQ ID NO: 25.

In some embodiments of the present invention, the amino acid sequence of the heavy chain variable region of the anti-TL1A antibody or antigen-binding fragment thereof is as shown in SEQ ID NO: 3, and the amino acid sequence of the light chain variable region is as shown in SEQ ID NO: 5.

In some embodiments of the present invention, the amino acid sequence of the heavy chain variable region of the anti-TL1A antibody or antigen-binding fragment thereof is as shown in SEQ ID NO: 16, and the amino acid sequence of the light chain variable region is as shown in SEQ ID NO: 17.

In some embodiments of the present invention, the amino acid sequence of the heavy chain variable region of the anti-TL1A antibody or antigen-binding fragment thereof is as shown in SEQ ID NO: 7, and the amino acid sequence of the light chain variable region is as shown in SEQ ID NO: 9.

In some embodiments of the present invention, the amino acid sequence of the heavy chain variable region of the anti-TL1A antibody or antigen-binding fragment thereof is as shown in SEQ ID NO: 24, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 26, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 27, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 28, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 29, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 30, and the amino acid sequence of the light chain variable region is as shown in SEQ ID NO: 25.

In some embodiments of the present invention, the amino acid sequence of the heavy chain variable region of the anti-TL1A antibody or antigen-binding fragment thereof is as shown in SEQ ID NO: 31, and the amino acid sequence of the light chain variable region is as shown in SEQ ID NO: 25.

In some embodiments of the present invention, the anti-TL1A antibody or antigen-binding fragment thereof comprises a monomer, a bivalent antibody, and/or a multivalent antibody.

In some embodiments of the present invention, the antigen-binding fragment is selected from the group consisting of scFv, Fab, Fab', F(ab')₂, Fv fragment, heavy chain antibody, and disulfide-linked Fv (dsFv).

In some embodiments of the present invention, the heavy chain constant region of the anti-TL1A antibody or antigen-binding fragment thereof is selected from the group consisting of human IgG1, IgG2, IgG3, or IgG4 heavy chain constant regions; preferably, it is the human IgG1 heavy chain constant region.

In some embodiments of the present invention, the light chain constant region of the anti-TL1A antibody or antigen-binding fragment thereof is selected from the group consisting of constant regions of human antibody κ chain or λ chain; preferably, it is the κ chain constant region.

The amino acid sequence of the heavy chain variable region or light chain variable region of the anti-TL1A antibody or antigen-binding fragment thereof further includes a derivative sequence that optionally undergoes addition, deletion, modification, and/or substitution of at least one amino acid in the aforementioned sequence and is capable of retaining TL1A binding affinity. In another preferred embodiment, the derivative sequence undergoes addition, deletion, modification, and/or substitution of 1 to 5 amino acids, such as 1, 2, or 3 amino acids, in any of the aforementioned amino acid sequences, and such that the derivative antibody comprising the VH and VL containing the derivative CDR sequences is capable of retaining TL1A binding affinity.

The anti-TL1A antibody or antigen-binding fragment thereof is selected from the group consisting of a full-length antibody, a single-chain antibody, or an antibody fragment.

A full-length antibody comprises a variable region (V) and a constant region (C). The constant region comprises a light chain constant region (LC) and a heavy chain constant region (HC). The constant region may be a constant region of a natural sequence or a variant thereof in amino acid sequence. The constant region of a natural sequence is, for example, a constant region of a natural sequence from a mammal, such as a human. In some embodiments of the present invention, the heavy chain constant region is an IgG1 constant region, and/or the light chain constant region is a kappa chain constant region (i.e., κ chain constant region). In some embodiments of the present invention, the amino acid sequence of the heavy chain constant region is as shown in SEQ ID NO: 33, and/or the amino acid sequence of the light chain constant region is as shown in SEQ ID NO: 34.

"Antibody fragment" comprises a portion of a full-length antibody, preferably comprising the antigen-binding region or variable region thereof. For example, antibody fragments include Fab, Fab', F(ab'), F(ab')₂, and Fv fragments. An Fv fragment is an antibody fragment that contains a complete antigen recognition and binding site. This region consists of a heavy chain variable region and a light chain variable region that are closely linked to each other, and this linkage may be covalent (such as in scFv). In such a conformation, the three CDRs of each variable region interact to define the antigen-binding site on the surface of the V-V dimer. The "Fab" fragment comprises the variable region and constant region of the light chain, and the variable region and first constant region (CH1) of the heavy chain. An F(ab') antibody fragment comprises a pair of Fab fragments that are typically covalently linked near the carboxy terminus via hinge cysteines between them.

The present invention also provides a recombinant protein, wherein the recombinant protein comprises: (i) the anti-TL1A antibody or antigen-binding fragment thereof; and (ii) optionally a tag sequence that facilitates expression and/or purification.

In some embodiments of the present invention, the tag sequence comprises an Fc tag, HA tag, GGGS sequence, FLAG tag, Myc tag, 6His tag, or a combination thereof.

In some embodiments of the present invention, the recombinant protein comprises a fusion protein.

In some embodiments of the present invention, the recombinant protein is a monomer, dimer, or multimer.

The present invention also provides an isolated polynucleotide which encodes the heavy chain variable region and/or light chain variable region or full-length amino acid sequence of anti-TL1A antibody or antigen-binding fragment thereof, or encodes the recombinant protein.

In some embodiments of the present invention, the polynucleotide is selected from RNA (e.g., mRNA) or DNA (e.g., cDNA).

In some embodiments of the present invention, the polynucleotide sequence encoding the heavy chain variable region of the anti-TL1A antibody or antigen-binding fragment thereof is as shown in SEQ ID NO: 2, SEQ ID NO: 6, SEQ ID NO: 35, SEQ ID NO: 37, or SEQ ID NO: 43-48.

In some embodiments of the present invention, the polynucleotide sequence encoding the light chain variable region of the anti-TL1A antibody or antigen-binding fragment thereof is as shown in SEQ ID NO: 4, SEQ ID NO: 8, SEQ ID NO: 36, or SEQ ID NO: 38.

The present invention also provides a nucleic acid construct comprising the isolated polynucleotide.

In some embodiments of the present invention, the nucleic acid construct is selected from the group consisting of: DNA, RNA, viral vectors, plasmids, transposons, other gene transfer systems, or combinations thereof.

In some embodiments of the present invention, the nucleic acid construct comprises a viral vector, such as lentivirus, adenovirus, AAV virus, retrovirus, or combinations thereof.

In some embodiments of the present invention, the nucleic acid construct is a plasmid, retrovirus, or lentiviral vector.

In some embodiments of the present invention, the nucleic acid construct is selected from the group consisting of: pTomo lentiviral vector, plenti, pLVTH, pLJM1, pHCMV, pLBS.CAG, pHR, pLV, and the like.

In some embodiments of the present invention, the nucleic acid construct further comprises elements selected from the group consisting of: promoters, the transcriptional enhancer element WPRE, long terminal repeats (LTRs), and the like. The term "nucleic acid construct" refers to an artificially constructed nucleic acid segment that can be introduced into target cells or tissues, and the nucleic acid construct may be various expression vectors, which include a vector backbone, i.e., an empty vector, and an expression cassette. The term "expression cassette" refers to a sequence having the potential to encode a protein.

The types of expression vectors are not specifically limited. An expression vector refers to a nucleic acid molecule that allows the insertion of exogenous nucleotides without disrupting the vector's ability to replicate and/or integrate in a host cell. The expression vector may include nucleic acid sequences that allow its replication in a host cell, such as an origin of replication. The expression vector may also include one or more selectable marker genes and other genetic elements. An expression vector is a vector containing the necessary regulatory sequences to enable transcription and translation of one or more inserted genes. The expression vector is selected from a eukaryotic expression vector or a prokaryotic expression vector.

The prokaryotic expression vector is selected from an E. coli expression vector, a Bacillus subtilis expression vector, or a Streptomyces expression vector. In a preferred embodiment, the prokaryotic expression vector is selected from an E. coli expression vector. Compared with other expression systems, the E. coli expression system has a well-characterized genetic background, short culture cycle, high expression level of the target gene, and strong resistance to contamination. The E. coli expression vector is, for example, a pET expression vector, specifically pET28a or pET32a, which can be stably expressed in E. coli. The expression vector may also be a pCW expression vector or a pUC expression vector.

The eukaryotic expression vector is selected from a yeast expression vector, an insect expression vector, or a mammalian expression vector. The mammalian expression vector is selected from a retroviral expression vector, a lentiviral expression vector, an adenoviral expression vector, or an adeno-associated viral expression vector. In a preferred embodiment, the eukaryotic expression vector is selected from a retroviral expression vector, wherein the retroviral expression vector can be stably expressed in cell lines, and the retroviral vector is, for example, pMSCV.

The host cell is selected from a eukaryotic host cell or a prokaryotic host cell. The eukaryotic host cell is selected from fungi such as yeast, insects, birds, plants, *Caenorhabditis elegans* (C. elegans) or nematodes, or mammalian host cells. A nonlimiting example of an insect cell is a *Spodoptera frugiperda* (Sf) cell. Examples of yeast host cells are *Saccharomyces cerevisiae* (S. cerevisiae), *Kluyveromyces lactis* (K. lactis), or *Yarrowia lipolytica.* Examples of mammalian cells are COS cells, baby hamster kidney cells, mouse L cells, LNCaP cells, Chinese hamster ovary (CHO) cells, human embryonic kidney (HEK) cells, African green monkey cells, CV1 cells, Vero cells, or Hep-2 cells. Examples of prokaryotic host cells include bacterial cells, such as *Escherichia coli, Streptomyces, Bacillus subtilis* (B. subtilis), *Salmonella typhi,* or mycobacteria.

A person skilled in the art can transfect the expression vector into a host cell according to methods well known in the art to obtain a cell comprising a coding gene for an anti-TL1A antibody or an antigen-binding fragment thereof. For example, introducing the expression vector into a eukaryotic cell can be performed by calcium phosphate co-precipitation, electroporation, microinjection, liposome transfection, or transfection using polyamine transfection reagents.

The present invention also provides a cell comprising the construct or having the exogenous polynucleotide integrated into its genome.

The cell of the present invention is obtained by transforming the nucleic acid construct into a host cell.

In some embodiments of the present invention, the cell includes a prokaryotic cell or a eukaryotic cell.

In some embodiments of the present invention, the cell is selected from the group consisting of: Escherichia coli, yeast cells, mammalian cells.

The present invention also provides a method for preparing the anti-TL1A antibody or antigen-binding fragment thereof, comprising the step of culturing the cell under conditions suitable for expressing the TL1A antibody or antigen-binding fragment thereof, thereby expressing the TL1A antibody or antigen-binding fragment thereof; further, the method also comprises the step of purifying and/or isolating the TL1A antibody or antigen-binding fragment thereof.

The present invention also provides a use of the anti-TL1A antibody or antigen-binding fragment thereof or the recombinant protein in the preparation of a medicament for treating a disease or in the preparation of a medicament for diagnosing a disease.

In the present invention, the disease is selected from one or more of inflammatory bowel disease, gastrointestinal disease associated with cystic fibrosis, colitis, irritable bowel syndrome, eosinophilic esophagitis, atopic dermatitis, eczema, scleroderma, arthritis or rheumatoid arthritis. Wherein the inflammatory bowel disease is Crohn's disease or ulcerative colitis.

The present invention also provides a pharmaceutical composition, the pharmaceutical composition comprising the anti-TL1A antibody or antigen-binding fragment thereof or the recombinant protein of the present invention.

In some embodiments of the present invention, the pharmaceutical composition comprises the anti-TL1A antibody or antigen-binding fragment thereof or the recombinant protein of the present invention, and a pharmaceutically acceptable carrier or excipient.

"Pharmaceutically acceptable" means that when the medicament is appropriately administered to an animal or human, it does not produce adverse, allergic, or other untoward reactions.

"Pharmaceutically acceptable carrier or excipient" shall be compatible with the active ingredient, meaning it can be blended therewith without substantially reducing the drug's efficacy under ordinary conditions. Specific examples of substances that may serve as pharmaceutically acceptable carriers or excipients include sugars such as lactose, glucose, and sucrose; starches such as corn starch and potato starch; cellulose and its derivatives such as sodium carboxymethyl cellulose, ethyl cellulose, and methyl cellulose; powdered tragacanth; malt; gelatin; talc; solid lubricants such as stearic acid and magnesium stearate; calcium sulfate; vegetable oils such as peanut oil, cottonseed oil, sesame oil, olive oil, corn oil, and cocoa butter; polyols such as propylene glycol, glycerol, sorbitol, mannitol, and polyethylene glycol; alginic acid; emulsifiers such as Tween; wetting agents such as sodium lauryl sulfate; coloring agents; flavoring agents; tableting agents; stabilizers; antioxidants; preservatives; pyrogen-free water; isotonic saline; and phosphate buffers. These substances are used as needed to help stabilize the formulation or to assist in enhancing activity or its bioavailability or, in the case of oral administration, to produce an acceptable taste or odor.

The form of the pharmaceutical composition is not particularly limited and may be various material forms such as solid, liquid, gel, semi-fluid, aerosol, etc.

The pharmaceutical composition is primarily intended for mammals. The mammal is preferably selected from rodents, artiodactyls, perissodactyls, lagomorphs, primates, etc. The primate is preferably a monkey, ape, or human.

The formulated pharmaceutical composition may be administered via conventional routes, including but not limited to: intravenous injection, intravenous infusion, subcutaneous injection, local injection, intramuscular injection, intratumoral injection, intraperitoneal injection (e.g., intraperitoneally), intracranial injection, or intracavitary injection.

The present invention also provides a method for treating a disease, the method comprising administering to a subject in need thereof the anti-TL1A antibody or antigen-binding fragment thereof, the recombinant protein, or the pharmaceutical composition of the present invention.

In certain embodiments of the present invention, the subject comprises a mammal, such as a human.

In certain embodiments of the present invention, the disease is selected from one or more of inflammatory bowel disease, gastrointestinal disease associated with cystic fibrosis, colitis, irritable bowel syndrome, eosinophilic esophagitis, atopic dermatitis, eczema, scleroderma, arthritis, or rheumatoid arthritis. Wherein the inflammatory bowel disease is Crohn's disease or ulcerative colitis.

In certain embodiments of the present invention, the method may be used in combination with other treatment methods.

In certain embodiments of the present invention, the other treatment methods include chemotherapy, radiotherapy, targeted therapy, and the like.

When treating the disease, a safe and effective amount of the antibody or antigen-binding fragment thereof is administered to the subject. Wherein the safe and effective amount is generally at least about 10 µg/kg body weight, and in most cases does not exceed about 50 mg/kg body weight; preferably, the dosage is about 10 µg/kg body weight to 10 mg/kg body weight. Without doubt, the specific dose should also take factors such as the route of administration and the patient's health status into consideration, all of which fall within the area of expertise of skilled physicians.

The embodiments of the present invention are illustrated below by specific examples, and other advantages and effects of the present invention will be readily apparent to those skilled in the art from the disclosure herein. The present invention may also be embodied or applied through other different specific embodiments, and various modifications or changes may be made to the details in this specification based on different perspectives and applications without departing from the spirit of the present invention.

Before further describing the specific embodiments of the present invention, it should be understood that the protection scope of the present invention is not limited to the specific embodiments described below; it should also be understood that the terms used in the embodiments of the present invention are intended to describe specific embodiments and not to limit the protection scope of the present invention; in the specification and claims of the present invention, unless explicitly indicated otherwise in the context, the singular forms "a", "an", and "the" include plural forms. When a numerical range is provided in an embodiment, it should be understood that, unless otherwise stated in the present invention, both endpoints of the numerical range and any value between the two endpoints may be selected. Unless otherwise defined, all technical and scientific terms used in the present invention have the same meanings as commonly understood by those skilled in the technical field. In addition to the specific methods, equipment, and materials used in the embodiments, any methods, equipment, and materials similar or equivalent to those described in the embodiments of the present invention may be used to implement the present invention based on the understanding of the prior art by those skilled in the technical field and the disclosure of the present invention. Unless otherwise stated, percentages and parts are by weight percentage and weight parts.

### Example 1 Antigen Immunization of Animals and Preparation and Screening of Hybridomas

### 1.1 Preparation of Antigen Protein and Positive Control Antibody

The sequence of the human TL1A extracellular domain (TL1A-ECD) used as the antigen was obtained from the UniProt database (Entry: 095150), with the amino acid sequence shown in SEQ ID NO: 1. A 6×His tag was added to its N-terminus, and it was constructed into the pcDNA 3.4 expression vector. After transfection into HEK-293F cells and expression for 5 days, the cell culture supernatant was collected and purified to obtain TL1A-His protein. Similarly, the 6×His tag was replaced with the Fc sequence of human IgG1, and after transfection into HEK-293F cells and expression purification, TL1A-Fc protein was obtained. The sequence of the positive control antibody Pra023 was obtained from patent WO-2021081365-A1.

### SEQ ID NO: 1

LKGQEFAPSHQQVYAPLRADGDKPRAHLTVVRQTPTQHFKNQFPALHWEHELGLAFTKNRMN
YTNKFLLIPESGDYFIYSQVTFRGMTSECSEIRQAGRPNKPDSITVVITKVTDSYPEPTQLLMGTK
SVCEVGSNWFQPIYLGAMFSLQEGDKLMVNVSDISLVDYTKEDKTFFGAFLL

### 1.2 Mouse Immunization

Experimental Balb/c mice, female, 6 to 8 weeks old, were acclimated in the laboratory environment for one week after purchase under a 12/12-hour light/dark cycle, with temperatures of 20-25°C and humidity of 40%-60%. The human TL1A-His protein expressed in Example 1.1 was used as the immunogen to conventionally immunize Balb/c mice at 50 µg/mouse/0.2 mL via intraperitoneal injection (IP). Immunization was performed three times on days 0, 14, and 28, and blood was collected on days 21 and 35 to detect antibody titers in mouse serum by ELISA. After three immunizations, mice with high antibody titers in serum were selected and boosted with human TL1A-His protein at 50 µg/mouse/0.2 mL via intraperitoneal injection (IP). Three to four days later, the spleens of the mice were harvested for fusion experiments.

### 1.3 Preparation and Screening of Hybridoma Cells

Three to four days after the final immunization, mouse splenocytes were fused with mouse myeloma cells SP2/0 using a conventional hybridoma technique with PEG. The fused cells were uniformly suspended in complete medium, which consisted of RPMI1640-GLUMAX supplemented with 1% Penicillin-Streptomycin, 20% FBS (fetal bovine serum), and 1× HAT. The fused cells were plated at 4×10⁴ cells/200 µL/well across a total of 60 96-well culture plates for cultivation. After 7-12 days, the supernatant was harvested, and hybridoma wells positive for human TL1A binding activity were screened by ELISA.

The method for screening hybridoma wells positive for human TL1A binding activity by ELISA is as follows: dilute TL1A-His to 1 µg/mL with PBS buffer, add 100 µL/well to the plate, and incubate overnight at 4°C; the next day, discard the supernatant, add 5% skim milk and block at 37°C for 1 hour, wash the plate 3 times with PBST for later use; add the collected hybridoma supernatant sequentially to the blocked plate, 100 µL/well, and incubate at 37°C for 1h; wash the plate 3 times with PBST, add HRP-labeled goat anti-mouse IgG secondary antibody, and incubate at 37°C for 30min; after washing the plate 3 times with PBST, remove residual droplets as much as possible on absorbent paper, add 100 µL of TMB to each well, and incubate at room temperature (20±5°C) protected from light for 5min; add 50 µL of 2M H₂SO₄ stop solution to each well to terminate the substrate reaction, and read the OD value at 450 nm with a microplate reader to analyze the binding ability of the test antibody to the target antigen TL1A. The hybridoma cell lines obtained through screening and expansion in complete medium containing serum were centrifuged and replaced with serum-free culture medium Hybridoma-SFM medium to achieve a cell density of 1-2×10⁶/mL, cultured under 8% CO₂ at 37°C for 1 week, centrifuged to collect the culture supernatant, purified by Protein G affinity chromatography to obtain anti-human TL1A monoclonal antibody protein, and through screening, a total of 60 hybridoma cell lines were obtained. All antibodies obtained from the hybridoma cell lines in the present invention can be obtained by genetic engineering methods.

### 1.4 Binding Capacity of Murine Antibodies to Human TL1A-His Protein

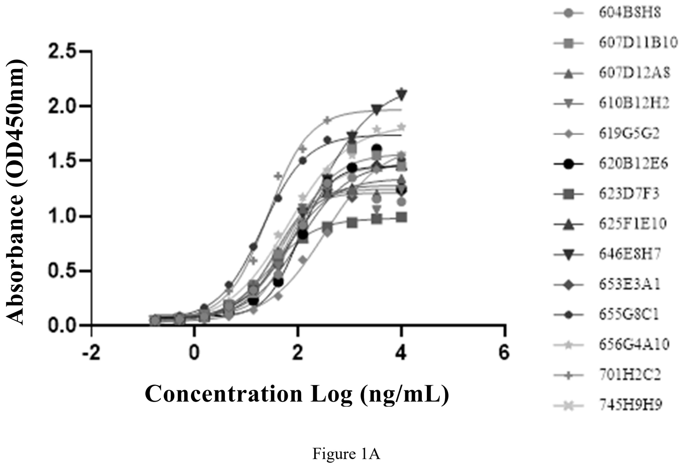

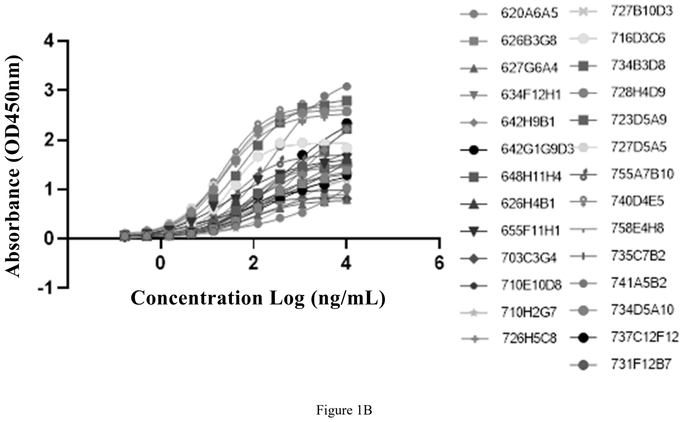

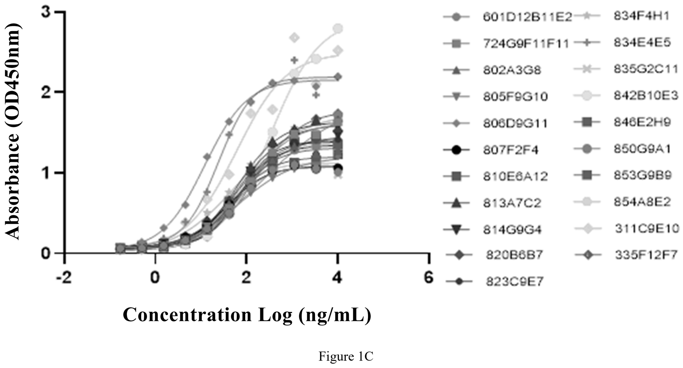

The binding capacity of murine antibodies to human TL1A-His protein was determined by enzyme-linked immunosorbent assay (ELISA). The specific method is as follows:
TL1A-His protein was diluted to 1 µg/mL with PBS buffer, added at 100 µL/well to the plate, and incubated overnight at 4°C; then blocked with 5% skim milk and incubated at 37°C for 1 hour; after washing the plate 3 times with PBST, the laboratory-prepared anti-human TL1A murine antibodies were serially diluted 3-fold from 10 µg/mL in 11 gradients using 1% BSA-PBS buffer, with 1% BSA-PBS as a blank control, added at 100 µL/well to the pre-coated TL1A-His plate, and incubated at 37°C for 1 hour; after washing the plate 3 times with PBST, HRP-labeled goat anti-mouse IgG secondary antibody was added and incubated at 37°C for 30 minutes; after washing the plate 3 times with PBST, residual droplets were removed as much as possible on absorbent paper, 100 µL of TMB was added to each well, and incubated at room temperature (20±5°C) protected from light for 5 minutes; 50 µL of 2M H₂SO₄ stop solution was added to each well to terminate the substrate reaction, and the OD value was read at 450 nm with a microplate reader to analyze the binding capacity of the test antibody to the antigen human TL1A-His. The obtained data were fitted and analyzed using GraphPad Prism 9 software, and the results are shown in Figures 1A, 1B, and 1C. As shown in Figures 1A, 1B, and 1C, most murine antibodies exhibited good binding activity to the target antigen TL1A-His, with EC₅₀ values as shown in Tables 1-1 to 1-3.

**Table 1-1**

| | | | | | | | |
|---|---|---|---|---|---|---|---|
| Murine-derived Antibody | 604B8H8 | 607D11B10 | 607D12A8 | 610B12H2 | 619G5G2 | 620B12E6 | 623D7F3 |
| EC₅₀ | 58.8 | 72.08 | 36.03 | 41.01 | 329.4 | 97.59 | 27.17 |
| Murine-derived Antibody | 625F1E10 | 646E8H7 | 653E3A1 | 655G8C1 | 656G4A10 | 701H2C2 | 745H9H9 |
| EC₅₀ | 103.8 | 187.3 | 58.46 | 20.68 | 64.62 | 26.34 | 50.51 |

**Table 1-2**

| | | | | | | | | | |
|---|---|---|---|---|---|---|---|---|---|
| Muri nederiv ed Antib ody | 620A6A5 | 626B3G8 | 627G6A 4 | 634F12H1 | 642H9B1 | 642G1G9D 3 | 648H11H 4 | 626H4B1 | 655F11H1 |
| EC₅₀ | 4.368×10¹⁵ | 177.6 | 85.3 | 124.7 | 1779 | 224.8 | 906.4 | 2.38×10²¹ | 33.11 |
| Muri ne-deriv ed Antib ody | 703C3G4 | 710E10D8 | 710H2G 7 | 726H5C8 | 727B10D 3 | 716D3C6 | 734B3D8 | 728H4D9 | 723D5A9 |
| EC₅₀ | 31.25 | 48.11 | 556.4 | 22.51 | 115.8 | 32.87 | 415.5 | 24.92 | 51.63 |
| Muri ne-deriv ed Antib ody | 727D5A5 | 755A7B1 0 | 740D4E 5 | 758E4H8 | 735C7B2 | 741A5B2 | 734D5A1 0 | 737C12F1 2 | 731F12B7 |
| EC₅₀ | 24.54 | 36.95 | 21.91 | 79.33 | 270.4 | 267.2 | 75.42 | 462.2 | 113.7 |

**Table 1-3**

| | | | | | | | |
|---|---|---|---|---|---|---|---|
| Murine-derived Antibody | 601D12B11E2 | 724G9F11F11 | 802A3G8 | 805F9G10 | 806D9G11 | 807F2F4 | 810E6A12 |
| EC₅₀ | 46.61 | 97.11 | 57.69 | 67.32 | 12.18 | 34.94 | 49.31 |
| Murine-derived Antibody | 823C9E7 | 834F4H1 | 834E4E5 | 835G2C11 | 842B10E3 | 846E2H9 | 850G9A1 |
| EC₅₀ | 72.55 | 64.84 | 24.17 | 39.38 | 335.4 | 76.58 | 236.5 |
| Murine-derived Antibody | 335F12F7 | 820B6B7 | 311C9E10 | 814G9G4 | 854A8E2 | 813A7C2 | 853G9B9 |
| EC₅₀ | 143.9 | 71.32 | 60.54 | 52.02 | 71.64 | 64.39 | 78.85 |

### 1.5 Determination of Blocking Activity of Murine Antibody Against the Binding of TL1A Protein to Stable Transfection Cell Line 293FT-DR3

This example employed fluorescence activated cell sorting (FACS) to determine the blocking activity of murine antibody against the binding of human TL1A protein to 293FT-DR3.

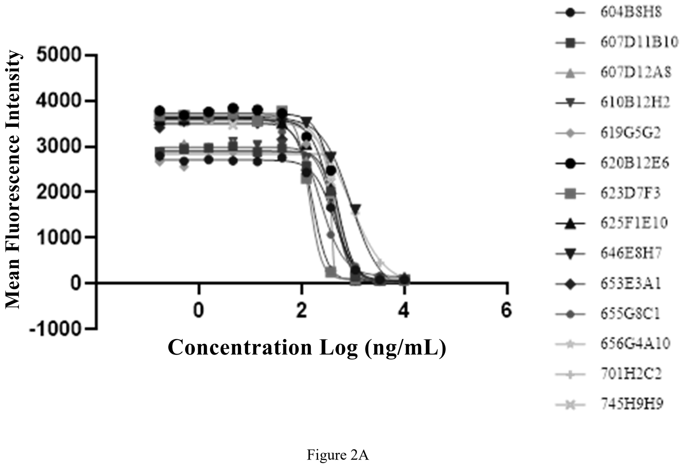

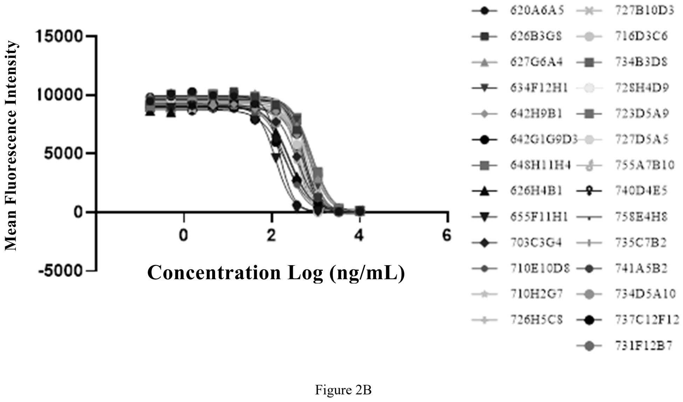

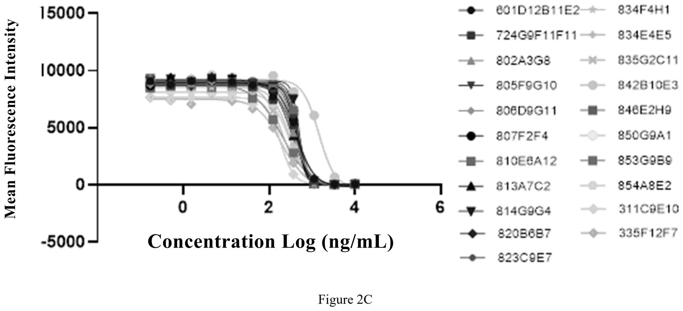

In this experiment, 293FT-DR3 (an engineered cell line constructed in the laboratory using lentiviral vectors to highly express human DR3) was used as target cells. TL1A was labeled with 647 fluorescent dye via Alexa Fluor^{™} 647 NHS Ester reagent (thermofisher). The 293FT-DR3 cells were counted and washed once with 1% BSA-PBS. Cells were plated at 2×10⁵/well with 100 µL/well, centrifuged to remove supernatant. Purified murine antibodies were serially diluted 3-fold for 11 gradients from 12 µg/mL using 1% BSA-PBS, with 1% BSA-PBS as blank control. 100 µL of each antibody dilution was mixed with TL1A-his-647 fluorescent protein (self-prepared, final concentration 100 ng/mL, 20 µL/well) and incubated at 37°C for 30 minutes. Then 100 µL of the mixture was taken to resuspend the 293FT-DR3 cell wells, followed by incubation at 4°C for 1 hour. After washing the cells twice with 1% BSA-PBS, the cells were resuspended in 200 µL PBS. The blocking activity of murine antibody against the binding of TL1A-his protein to the cells was determined by flow cytometry. The obtained data were fitted and analyzed using GraphPad Prism 9 software, and the results are shown in Figures 2A, 2B, and 2C. The results indicate that most murine antibodies can specifically block the binding of DR3 on the surface of 293FT cells to TL1A-his protein, with IC₅₀ values as shown in Tables 2-1 to 2-3.

**Table 2-1**

| | | | | | | | |
|---|---|---|---|---|---|---|---|
| Murine-derived Antibody | 604B8H8 | 607D11B10 | 607D12A8 | 610B12H2 | 619G5G2 | 620B12E6 | 623D7F3 |
| IC₅₀ | 436.1 | 471.1 | 462.1 | 437.7 | 378.7 | 463.3 | 140.9 |
| Murine-derived Antibody | 625F1E10 | 646E8H7 | 653E3A1 | 655G8C1 | 656G4A10 | 701H2C2 | 745H9H9 |
| IC₅₀ | 392.4 | 917.5 | 162.5 | 219.1 | 460 | 871.8 | 436 |

**Table 2-2**

| | | | | | | | | | |
|---|---|---|---|---|---|---|---|---|---|
| Muri ne-deriv ed Antib ody | 620A6A 5 | 626B3G8 | 627G6A 4 | 634F12H1 | 642H9B1 | 642G1G9D 3 | 648H11H 4 | 626H4B1 | 655F11H1 |
| IC₅₀ | 149.6 | 520.4 | 675.9 | 683.8 | 516.6 | 499.5 | 821.1 | 239.1 | 121.9 |
| Muri ne-deriv ed Antib ody | 703C3G 4 | 710E10D8 | 710H2G 7 | 726H5C8 | 727B10D 3 | 716D3C6 | 734B3D8 | 728H4D9 | 723D5A9 |
| IC₅₀ | 367.4 | 209.7 | 742.3 | 540.8 | 633.3 | 422.9 | 816.4 | 445.3 | 760.6 |
| Muri ne-deriv ed Antib ody | 727D5A 5 | 755A7B1 0 | 740D4E 5 | 758E4H8 | 735C7B2 | 741A5B2 | 734D5A1 0 | 737C12F1 2 | 731F12B7 |
| IC₅₀ | 427.1 | 361.6 | 419.1 | 254.5 | 461 | 503 | 430.1 | 209.4 | 554.7 |

**Table 2-3**

| | | | | | | | |
|---|---|---|---|---|---|---|---|
| Murine-derived Antibody | 601D12B11E2 | 724G9F11F11 | 802A3G8 | 805F9G10 | 806D9G11 | 807F2F4 | 810E6A12 |
| IC₅₀ | 433.8 | 402.2 | 353.9 | 444.2 | 398.8 | 435.3 | 488.9 |
| Murine-derived Antibody | 823C9E7 | 834F4H1 | 834E4E5 | 835G2C11 | 842B10E3 | 846E2H9 | 850G9A1 |
| IC₅₀ | 433 | 298.7 | 415.3 | 208.6 | 1434 | 460.3 | 463.4 |
| Murine-derived Antibody | 335F12F7 | 820B6B7 | 311C9E10 | 813A7C2 | 853G9B9 | 814G9G4 | 854A8E2 |
| IC₅₀ | 186.1 | 454.9 | 179.8 | 332.9 | 199.3 | 501.6 | 297.6 |

### Example 2 Humanization of Murine Anti-Human TL1A Monoclonal Antibody 2.1 Determination of Variable Region Sequences of Murine Anti-Human TL1A Monoclonal Antibody

Hybridoma clones 728H4D9 and 311C9E10 were selected as candidate antibodies. Total RNA was extracted from the corresponding hybridoma monoclonal cell lines using Trizol (purchased from Life Technologies), and mRNA was reverse transcribed into cDNA using a reverse transcription kit (purchased from Takara). PCR was performed using combinatorial primers reported in the literature (Antibody Engineering, Volume 1, Edited by Roland Kontermann and Stefan Dübel, primer sequences from page 323). The obtained PCR products were sequenced and analyzed via the Kabat database to confirm that the obtained sequences were variable region sequences of murine antibodies. The heavy chain variable region gene sequence of 728H4D9 has a total length of 357 bp, encoding 119 amino acid residues, with the nucleotide sequence as shown in SEQ ID NO: 2 and the amino acid sequence as shown in SEQ ID NO: 3. The light chain variable region gene sequence has a total length of 318 bp, encoding 106 amino acid residues, with the nucleotide sequence as shown in SEQ ID NO: 4 and the amino acid sequence as shown in SEQ ID NO: 5. The heavy chain variable region gene sequence of 311C9E10 has a total length of 357 bp, encoding 119 amino acid residues, with the nucleotide sequence as shown in SEQ ID NO: 6 and the amino acid sequence as shown in SEQ ID NO: 7; the light chain variable region gene sequence has a total length of 318 bp, encoding 106 amino acid residues, with the nucleotide sequence as shown in SEQ ID NO: 8 and the amino acid sequence as shown in SEQ ID NO: 9.
SEQ ID NO: 2 
GAGGTCCAGCTGCAACAGTCTGGACCTGAGCTGGTGAAGCCTGGAGCTTCAATGAAGATAT
CCTGCAAGGCTTCTGGTTACTCATTCACTGGCTACACCATGAACTGGGTGAAGCAGAGCCAT
GGAAAGAACCTTGAGTGGATTGGACTTATTAATCCTTACAGCGGTGGTACTAACTACAACC
 
AGAAGTTCAAGGGCAAGGCCACATTAACTGTAGACAAGACATCCAGCACAGCCTACATGGA
GCTCCTCAGTCTGACATCTGAGGACTCTGCAGTCTATTACTGTGCGTCCATCTACCAAAGGC
ACGACGGGATTGCTTACTGGGGCCAAGGGACTCTGGTCACTGTCTCTGCA
SEQ ID NO: 3 
EVQLQQSGPELVKPGASMKISCKASGYSFTGYTMNWVKQSHGKNLEWIGLINPYSGGTNYNQK
FKGKATLTVDKTSSTAYMELLSLTSEDSAVYYCASIYQRHDGIAYWGQGTLVTVSA
SEQ ID NO: 4 
CAAATTGTTCTCACCCAGTCTCCAGCAATCATGTCTGCATCTCCAGGGGAGAAGGTCACCAT
GACCTGCAGTGCCAGCTCAAGTGTAAATTACATGCACTGGTACCAGCAGAAGTCAGGCACC
TCCCCCAAAAGATGGATTTATGACACATCCAAACTGGCTTCTGGAGTCCCAACTCGCTTCAG
TGGCAGTGGGTCTGGGACCTCTTACTCTCTCACAATCAGCAGCATGGAGGCTGAAGATGCT
GCCACTTATTACTGCCAGCAGTGGAGTAGTAGCCCATACACGTTCGGAGGGGGGACCAAGC
TGGAAATCAAA
SEQ ID NO: 5 
QIVLTQSPAIMSASPGEKVTMTCSASSSVNYMHWYQQKSGTSPKRWIYDTSKLASGVPTRFSGS
GSGTSYSLTISSMEAEDAATYYCQQWSSSPYTFGGGTKLEIK
SEQ ID NO: 6 
GAAGTGAAACTTGAGGAGTCTGGAGGAGGCTTAGTGCAACCTGGAGGATCCATGAAACTCT
CCTGTGTTGCCTCTGGATTCACTTTCAGTAACTACTGGATGAACTGGGTCCGCCAGTCTCCA
GAGAAGGGGCTTGAGTGGGTTGCTGGAATTAGATTGAAATCTAATAATTATACAACACAGT
ATGCGGAGTCTGTGAAAGGGAGGTTCACCATTTCAAGAGATGATTCCAAAAGTAGTGTCTA
CCTGCAAATGAACAACTTAAGAGCTGAAGACACTGGCATTTATTATTGTACCCCATTACTCC
TCAATGGTATGGACTACTGGGGTCAAGGAACCTCAGTCACCGTCTCCTCA
SEQ ID NO: 7 
EVKLEESGGGLVQPGGSMKLSCVASGFTFSNYWMNWVRQSPEKGLEWVAGIRLKSNNYTTQY
AESVKGRFTISRDDSKSSVYLQMNNLRAEDTGIYYCTPLLLNGMDYWGQGTSVTVSS
SEQ ID NO: 8 
GAAAATGTTCTCACCCAGTCTCCAGCAATCATGTCTGCATCTCCAGGGGAAAAGGTCATCAT
GACCTGTAGTGCCAGCTCAAGTGTAAGCTACATGCACTGGTACCAGCGGAAGTCAAACACC
TCCCCCAAACTCTGGATTTATGACACATCCAATCTGGCTTCTGGAGTCCCAGGTCGCTTCAG
TGGCACTGGGTCTGGAAACTCTTACTCTCTCACGATCAGCAGCATGGAGGCTGAAGATGTTG
CCACTTATTACTGTTTTCAGGAGAGTGGGTACCCATTCACGTTCGGCTCGGGGACAAAGTTG
GAAATAAAA
SEQ ID NO: 9

ENVLTQSPAIMSASPGEKVIMTCSASSSVSYMHWYQRKSNTSPKLWIYDTSNLASGVPGRFSGT

GSGNSYSLTISSMEAEDVATYYCFQESGYPFTFGSGTKLEIK

### 2.2 Humanization of Murine Anti-Human TL1A Monoclonal Antibody

The amino acid sequences of the heavy and light chain variable regions of 728H4D9 were analyzed, and three antigen complementarity determining regions (CDRs) and four framework regions (FRs) were determined according to Kabat rules. Among them, the amino acid sequences of the heavy chain complementarity determining regions are HCDR1: GYTMN (SEQ ID NO: 10), HCDR2: LINPYSGGTNYNQKFKG (SEQ ID NO: 11), and HCDR3: IYQRHDGIAY (SEQ ID NO: 12); the amino acid sequences of the light chain complementarity determining regions are LCDR1: SASSSVNYMH (SEQ ID NO: 13), LCDR2: DTSKLAS (SEQ ID NO: 14), and LCDR3: QQWSSSPYT (SEQ ID NO: 15).

By performing homology comparison with human IgG germline sequences (Germline) in NCBI IgBlast, IGHV1-2*02 was selected as the heavy chain CDR grafting template and IGKV6-21*01 as the light chain CDR grafting template. The CDR regions of the 728H4D9 antibody were grafted onto the selected humanized templates, replacing the CDR regions of the human templates. The heavy chain variable region was then recombined with the human IgG1 constant region (amino acid sequence as shown in SEQ ID NO: 33), and the light chain variable region was recombined with the human kappa chain constant region (amino acid sequence as shown in SEQ ID NO: 34). Based on the three-dimensional structure of the antibody, back mutations were performed on buried residues, residues directly interacting with the CDR regions, and residues significantly influencing the conformation of the antibody's VL and VH. Multiple humanized antibodies were obtained, and through affinity screening, the amino acid sequence of the heavy chain variable region (SEQ ID NO: 16) and the light chain variable region (SEQ ID NO: 17) of the humanized antibody 72851 were determined, with nucleotide sequences shown in SEQ ID NO: 35 and SEQ ID NO: 36, respectively.
SEQ ID NO: 16 
QVQLVQSGAEVKKPGASVKVSCKASGYSFTGYTMNWVRQAPGQNLEWIGLINPYSGGTNYNQ
KFKGRATLTVDTSISTAYMELSRLRSDDTAVYYCASIYQRHDGIAYWGQGTLVTVSS
SEQ ID NO: 17 
EIVLTQSPDFQSVTPKEKVTITCSASSSVNYMHWYQQKPDQSPKLLIKDTSKLASGVPSRFSGSG
SGTDFTLTINSLEAEDAATYYCQQWSSSPYTFGGGTKVEIK
SEQ ID NO: 33 ASTKGPSVFPLAPSSKSTSGGTAALGCLVKDYFPEPVTVSWNSGALTSGVHTFPAVLQSSGLYSL 
SSVVTVPSSSLGTQTYICNVNHKPSNTKVDKKVEPKSCDKTHTCPPCPAPEAAGGPSVFLFPPKP
KDTLYITREPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHEALHNHYT
QKSLSLSPGK
SEQ ID NO: 34
SEQ ID NO: 35 
CAAGTGCAGCTGGTGCAGAGCGGCGCCGAGGTGAAGAAGCCTGGCGCTAGCGTGAAGGTG
AGCTGCAAGGCTAGCGGCTACAGCTTCACCGGCTACACCATGAACTGGGTGAGACAAGCCC
CTGGACAGAACCTGGAGTGGATCGGCCTGATCAACCCTTACAGCGGCGGAACCAACTACAA
TCAGAAGTTCAAGGGCAGAGCCACACTGACCGTGGACACAAGCATCAGCACCGCCTACATG
GAGCTGAGCAGACTGAGAAGCGACGACACCGCCGTGTATTATTGCGCCTCCATCTATCAGA
GACACGACGGCATCGCCTACTGGGGCCAAGGCACCCTGGTGACCGTGAGCAGC
SEQ ID NO: 36

GAGATCGTGCTGACACAGAGCCCTGACTTTCAGAGCGTGACCCCTAAGGAGAAGGTGACCA
TTACCTGCAGCGCTAGCAGCAGCGTGAACTACATGCACTGGTATCAGCAGAAGCCTGATCA
GAGCCCTAAGCTGCTGATCAAGGACACAAGCAAGCTGGCTAGCGGCGTGCCTAGCAGATTC
AGCGGCAGCGGCAGCGGCACCGACTTCACCCTGACCATCAACAGCCTGGAGGCCGAGGAC
GCCGCCACCTACTACTGTCAGCAGTGGAGCAGCAGCCCTTACACCTTCGGCGGAGGCACCA
AGGTGGAGATCAAA

The amino acid sequences of the heavy and light chain variable regions of 311C9E10 were analyzed, and three antigen complementarity determining regions (CDRs) and four framework regions (FRs) were determined according to Kabat rules. Among them, the amino acid sequences of the heavy chain complementarity determining regions are HCDR1: NYWMN (SEQ ID NO: 18), HCDR2: GIRLKSNNYTTQYAESVKG (SEQ ID NO: 19), and HCDR3: LLLNGMDY (SEQ ID NO: 20); the amino acid sequences of the light chain complementarity determining regions are LCDR1: SASSSVSYMH (SEQ ID NO: 21), LCDR2: DTSNLAS (SEQ ID NO: 22), and LCDR3: FQESGYPFT (SEQ ID NO: 23).

By performing homology comparison with human IgG germline sequences (Germline) in NCBI IgBlast, IGHV3-73*01 was selected as the heavy chain CDR grafting template and IGKV6-21*01 as the light chain CDR grafting template. The CDR regions of the 311C9E10 antibody were grafted onto the selected humanized templates, replacing the CDR regions of the human templates. The heavy chain variable region was then recombined with the human IgG1 constant region (amino acid sequence as shown in SEQ ID NO: 33), and the light chain variable region was recombined with the human kappa chain constant region (amino acid sequence as shown in SEQ ID NO: 34). Based on the three-dimensional structure of the antibody, back mutations were performed on buried residues, residues directly interacting with the CDR regions, and residues significantly influencing the conformation of the antibody's VL and VH. Multiple humanized antibodies were obtained, and through affinity screening, the amino acid sequence of the heavy chain variable region (SEQ ID NO: 24) and the light chain variable region (SEQ ID NO: 25) of the humanized antibody 31122 were determined, with nucleotide sequences shown in SEQ ID NO: 37 and SEQ ID NO: 38, respectively.
SEQ ID NO: 24 
EVQLVESGGGLVQPGGSLKLSCAASGFTFSNYWMNWVRQASGKGLEWVGGIRLKSNNYTTQY
AESVKGRFTISRDDSKNTAYLQMNSLKTEDTAVYYCTPLLLNGMDYWGQGTTVTVSS
SEQ ID NO: 25 
EIVLTQSPDFQSVTPKEKVTITCSASSSVSYMHWYQQKPDQSPKLWIYDTSNLASGVPSRFSGSG
SGTDFTLTINSLEAEDAATYYCFQESGYPFTFGQGTKLEIK
SEQ ID NO: 37 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACACCACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAACGGCATGGACTACTGGGGCCAAGGCACCACCGTGACCGTGAGCAGC
SEQ ID NO: 38

GAGATCGTGCTGACACAGAGCCCTGACTTTCAGAGCGTGACCCCTAAGGAGAAGGTGACCA
TCACCTGCAGCGCTAGCAGCAGCGTGAGCTACATGCACTGGTATCAGCAGAAGCCTGATCA
GAGCCCTAAGCTGTGGATCTACGACACAAGCAACCTGGCTAGCGGCGTGCCTAGCAGATTC
AGCGGCAGCGGCAGCGGCACCGACTTCACCCTGACCATCAACAGCCTGGAGGCCGAGGAC
GCCGCCACCTACTACTGCTTCCAAGAGAGCGGCTACCCTTTCACCTTCGGCCAAGGCACCAA
GCTGGAGATCAAA

### Example 3 Determination of the Affinity Dissociation Constant KD of Humanized Antibodies

Using a Biacore 8K molecular interaction analyzer, the kinetic parameters of binding and dissociation between candidate humanized antibodies 72851, 31122, positive control antibody PRA023, and antigen TL1A-his were determined by capture method. Antibodies were diluted to 2 µg/mL with HBS-EP+, pH 7.4 buffer and captured on a Protein A chip. The antigen was diluted with HBS-EP+, pH 7.4 buffer and set at six concentration gradients with a maximum concentration of 50 nM for binding to antibodies, followed by dissociation in HBS-EP+, pH 7.4 buffer. Experimental results are shown in Table 3. The affinity of candidate humanized antibodies 72851 and 31122 for TL1A-his was superior to that of the positive antibody PRA023.

**Table 3: Affinity Dissociation Constants**

| Mobile Phase | Stationary Phase | KD (M) | ka (1/Ms) | kd (1/s) |
|---|---|---|---|---|
| TL1A-his | 72851 | 2.12E-10 | 1.00E+06 | 2.12E-04 |
| TL1A-his | 31122 | 3.94E-10 | 4.05E+05 | 1.59E-04 |
| TL1A-his | PRA023 | 5.37E-10 | 3.08E+05 | 1.66E-04 |

| | | | | |
|---|---|---|---|---|
| Note: KD is the affinity constant; ka is the association rate constant; kd is the dissociation rate constant. | | | | |

### Example 4 Optimization of Physicochemical Properties of Humanized Antibody 31122

Due to the high glycosylation modification and charge heterogeneity of humanized antibody 31122, a mutant library was constructed by introducing single-point saturation mutations and multiple superimposed beneficial mutations at potential glycosylation and deamidation sites in the CDR sequences of the heavy chain variable region (with the light chain and heavy chain constant region unchanged). Through ELISA affinity and capillary isoelectric focusing assays, candidate antibodies with both improved affinity and charge heterogeneity compared to the parent 31122 antibody were screened.

The CDR sequences of the heavy chain variable regions of the modified candidate antibodies are shown in Table 4.

**Table 4: CDR Amino Acid Sequences**

| | HCDR1 | HCDR2 | HCDR3 |
|---|---|---|---|
| 311V25 | NYWMN (SEQ ID NO: 18) | GIRLKSNNYATQYAESVKG (SEQ ID NO: 39) | LLLNGMDY (SEQ ID NO: 20) |
| 311V31 | NYWMN (SEQ ID NO: 18) | GIRLKSNNYKTQYAESVKG (SEQ ID NO: 40) | LLLNGMDY (SEQ ID NO: 20) |
| 311V34 | NYWMN (SEQ ID NO: 18) | GIRLKSNNYRTQYAESVKG (SEQ ID NO: 41) | LLLNGMDY (SEQ ID NO: 20) |
| 311V25-7 | NYWMN (SEQ ID NO: 18) | GIRLKSNNYATQYAESVKG (SEQ ID NO: 39) | LLLSGMDY (SEQ ID NO: 42) |
| 311V31-7 | NYWMN (SEQ ID NO: 18) | GIRLKSNNYKTQYAESVKG (SEQ ID NO: 40) | LLLSGMDY (SEQ ID NO: 42) |
| 311V34-7 | NYWMN (SEQ ID NO: 18) | GIRLKSNNYRTQYAESVKG (SEQ ID NO: 41) | LLLSGMDY (SEQ ID NO: 42) |

The amino acid sequences of the heavy chain variable regions of the modified candidate antibodies are shown in SEQ ID NO: 26 to 31, and the nucleotide sequences are shown in SEQ ID NO: 43 to 48, respectively.
311V25-HC (SEQ ID NO: 26) 
EVQLVESGGGLVQPGGSLKLSCAASGFTFSNYWMNWVRQASGKGLEWVGGIRLKSNNYATQY
AESVKGRFTISRDDSKNTAYLQMNSLKTEDTAVYYCTPLLLNGMDYWGQGTTVTVSS
311V31-HC (SEQ ID NO: 27) 
EVQLVESGGGLVQPGGSLKLSCAASGFTFSNYWMNWVRQASGKGLEWVGGIRLKSNNYKTQY
AESVKGRFTISRDDSKNTAYLQMNSLKTEDTAVYYCTPLLLNGMDYWGQGTTVTVSS
311V34-HC (SEQ ID NO: 28) 
EVQLVESGGGLVQPGGSLKLSCAASGFTFSNYWMNWVRQASGKGLEWVGGIRLKSNNYRTQY
AESVKGRFTISRDDSKNTAYLQMNSLKTEDTAVYYCTPLLLNGMDYWGQGTTVTVSS
311V25-7-HC (SEQ ID NO: 29) 
EVQLVESGGGLVQPGGSLKLSCAASGFTFSNYWMNWVRQASGKGLEWVGGIRLKSNNYATQY
AESVKGRFTISRDDSKNTAYLQMNSLKTEDTAVYYCTPLLLSGMDYWGQGTTVTVSS
311V31-7-HC (SEQ ID NO: 30) 
EVQLVESGGGLVQPGGSLKLSCAASGFTFSNYWMNWVRQASGKGLEWVGGIRLKSNNYKTQY
AESVKGRFTISRDDSKNTAYLQMNSLKTEDTAVYYCTPLLLSGMDYWGQGTTVTVSS
311V34-7-HC (SEQ ID NO: 31) 
EVQLVESGGGLVQPGGSLKLSCAASGFTFSNYWMNWVRQASGKGLEWVGGIRLKSNNYRTQY
AESVKGRFTISRDDSKNTAYLQMNSLKTEDTAVYYCTPLLLSGMDYWGQGTTVTVSS
SEQ ID NO: 43 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACGCCACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAACGGCATGGACTACTGGGGCCAAGGCACCACCGTGACCGTGAGCAGC
SEQ ID NO: 44 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACAAGACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAACGGCATGGACTACTGGGGCCAAGGCACCACCGTGACCGTGAGCAGC
SEQ ID NO: 45 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACAGAACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAACGGCATGGACTACTGGGGCCAAGGCACCACCGTGACCGTGAGCAGC
SEQ ID NO: 46 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACGCCACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAGCGGCATGGACTACTGGGGCCAAGGCACCACCGTGACCGTGAGCAGC
SEQ ID NO: 47 
GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACAAGACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAGCGGCATGGACTACTGGGGCCAAGGCACCACCGTGACCGTGAGCAGC
SEQ ID NO: 48

GAGGTGCAGCTGGTCGAGTCCGGAGGCGGACTGGTCCAACCTGGCGGAAGCCTGAAGCTGA
GCTGCGCCGCTAGCGGCTTCACCTTCAGCAACTACTGGATGAACTGGGTGAGACAAGCTAG
CGGCAAGGGCCTGGAGTGGGTGGGCGGCATCAGACTGAAGAGCAACAACTACAGAACACA
GTACGCCGAGAGCGTGAAGGGCAGATTCACCATCAGCAGAGACGACAGCAAGAACACCGC
CTACCTGCAGATGAACAGCCTGAAGACCGAGGACACCGCCGTGTACTACTGCACACCTCTG
CTCCTGAGCGGCATGGACTACTGGGGCCAAGGCACCACCGTGACCGTGAGCAGC

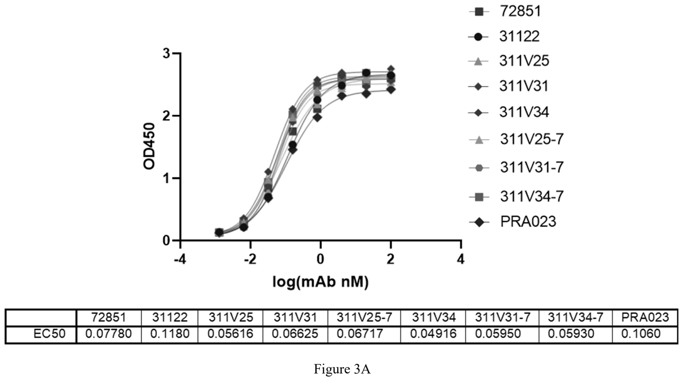

ELISA experimental results are shown in Figure 3A, indicating that the binding affinity of candidate antibodies 72851, 31122, 311V25, 311V31, 311V34, 311V25-7, 311V31-7, and 311V34-7 to antigen TL1A-his is superior to that of the positive control PRA023.

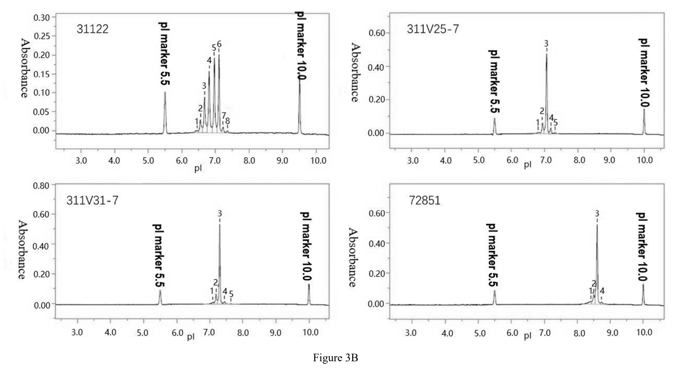

Capillary isoelectric focusing results of anti-TL1A candidate antibodies are shown in Figure 3B, demonstrating that the charge heterogeneity of modified antibodies 311V25-7 and 311V31-7 is improved compared to their parent antibody 31122 and is comparable to that of candidate antibody 72851.

### Example 5 Assay of Anti-TL1A Antibody Blocking TL1A Binding to DR3

This example employs fluorescence-activated cell sorting (FACS) to determine the blocking effect of anti-TL1A antibodies on the binding between TL1A and its cell surface receptor DR3. The target cells used in the experiment are laboratory-constructed 293FT engineered cell lines with high expression of human DR3 (293FT-DR3 cell line). The sequence of DR3 is derived from the UniProt database (Entry: Q93038), and its amino acid sequence is shown in SEQ ID NO: 32.

SEQ ID NO: 32
MEQRPRGCAAVAAALLLVLLGARAQGGTRSPRCDCAGDFHKKIGLFCCRGCPAGHYLKAPCTE
PCGNSTCLVCPQDTFLAWENHHNSECARCQACDEQASQVALENCSAVADTRCGCKPGWFVEC
QVSQCVSSSPFYCQPCLDCGALHRHTRLLCSRRDTDCGTCLPGFYEHGDGCVSCPTSTLGSCPE
RCAAVCGWRQMFWVQVLLAGLVVPLLLGATLTYTYRHCWPHKPLVTADEAGMEALTPPPAT
HLSPLDSAHTLLAPPDSSEKICTVQLVGNSWTPGYPETQEALCPQVTWSWDQLPSRALGPAAAP
TLSPESPAGSPAMMLQPGPQLYDVMDAVPARRWKEFVRTLGLREAEIEAVEVEIGRFRDQQYE
MLKRWRQQQPAGLGAVYAALERMGLDGCVEDLRSRLQRGP

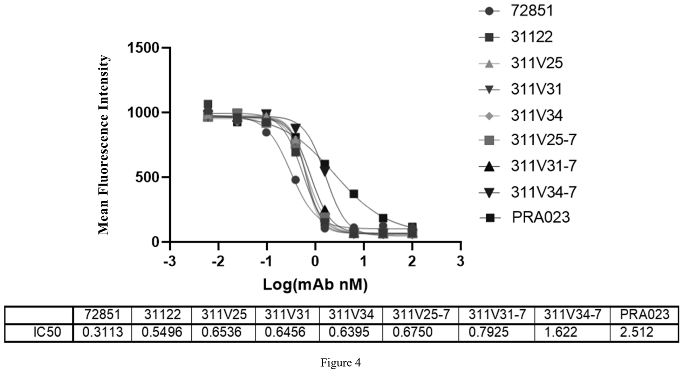

TL1A-his antigen was conjugated with Alexa Fluor^{™} 647 fluorescent dye (purchased from Invitrogen, Cat. No. A20006) for later use. The conjugated antigen TL1A-his-647 was diluted to 200 ng/mL with 1% BSA-PBS. Anti-TL1A antibodies were diluted to 200 nM with 1% BSA-PBS and subjected to 4-fold serial dilution to generate 8 concentration gradients. The 200 ng/mL TL1A-his-647 was added to the antibody solutions at different concentration gradients and incubated at 4°C for 1 hour. The mixture was then added to a 293FT-DR3 cell plate (1×10⁵ cells/well) from which the supernatant had been removed, followed by incubation at 4°C for 1 hour. The cells were washed twice with PBS to remove unbound antigen, and finally resuspended in 200 µL PBS. The mean fluorescence intensity was measured by flow cytometry, and the obtained data were fitted and analyzed using GraphPad Prism 9 software. The results are shown in Figure 4. Experimental results indicate that candidate antibodies 72851, 31122, 311V25, 311V31, 311V34, 311V25-7, 311V31-7, 311 V34-7, and the positive control antibody PRA023 all could block the binding of TL1A to DR3, and the blocking capacity of the candidate antibodies was superior to that of PRA023.

### Example 6 Assay of Anti-TL1A Antibody Inhibiting TLIA-Induced NF-κB Activation

After TL1A binds to its receptor DR3, it activates the intracellular NF-κB signaling pathway and triggers apoptosis. This example determines the inhibition of TL1A-induced NF-κB pathway activation by anti-TL1A antibodies by constructing a TF-1 cell line (TF-1-NF-κB-Luc cells) stably expressing luciferase under the regulation of NF-κB.

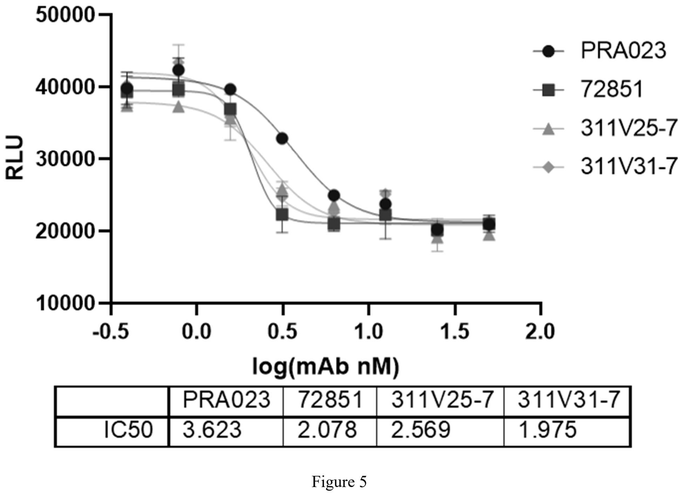

TL1A-his protein was diluted to 800 ng/mL (final concentration 200 ng/mL) with RPMI 1640+10% FBS medium and added to a flat-bottom 96-well plate, 25 µL per well. Anti-TL1A antibodies were diluted to 200 nM with RPMI 1640+10% FBS medium, subjected to 2-fold serial dilution to generate 8 concentration gradients, and 25 µL was added to the TL1A-his-containing wells, with the initial final concentration of anti-TL1A antibody being 50 nM. TF-1-NF-κB-Luc cells were centrifuged and counted, and the cells adjusted to the appropriate density were added to the aforementioned 96-well cell plate at 1×10⁵ cells/well, 50 µL per well, followed by incubation in a 37°C incubator for 24 hours. The next day, the cell plate was removed and allowed to equilibrate at room temperature for approximately 15 minutes. Then, 100 µL of Bio-Lite Luciferase (purchased from Nanjing Vazyme Biotech Co., Ltd., Cat. No.: DD1201-02) was added to each well. After incubation at room temperature for 10 minutes, the luminescence (RLU) value was read using a microplate reader. Data were analyzed with GraphPad Prism 9, and graphs were generated and IC₅₀ values were calculated. The results are shown in Figure 5. The experimental results indicate that candidate antibodies 72851, 311V25-7, 311V31-7, and the positive control antibody PRA023 all effectively inhibit TL1A-induced activation of NF-κB, with the inhibitory capacity of the candidate antibodies being superior to that of the positive control antibody PRA023.

### Example 7: Experiment of Anti-TL1A Antibody Inhibiting TL1A-Induced Apoptosis in TF-1 Cells

TL1A is an effective and specific inhibitor of endothelial cell growth, and it induces apoptosis through binding to DR3. This example employs fluorescence-activated cell sorting (FACS) to determine the inhibition of TL1A-induced apoptosis in TF-1 cells by Anti-TL1A antibodies.

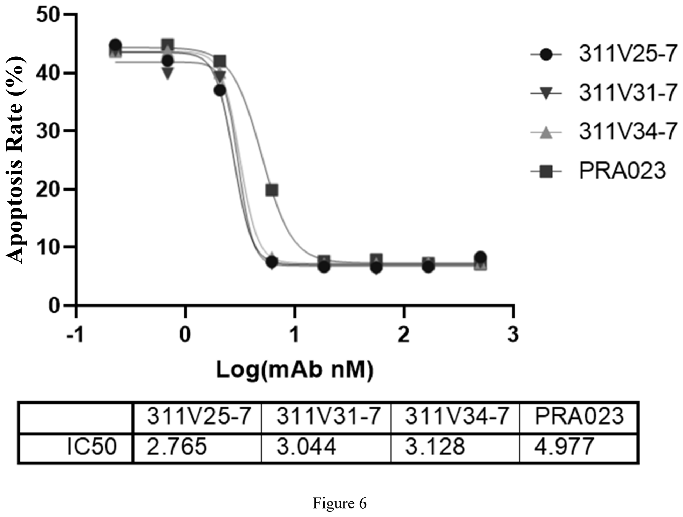

TF-1 cells were centrifuged and counted, and the cell density was adjusted and plated into a 3599 cell plate at 1×10⁵ cells/well, 100 µL/well. TL1A-his was diluted to 800 ng/mL using 1640 medium (containing 10% FBS and 1% PS), while cycloheximide (CHX, purchased from CST, Cat. No.: 2112) was added to the centrifuge tube at a final concentration of 40 µg/mL. After thorough mixing, 50 µL/well was added to the aforementioned 3599 cell plate. The positive control antibody PRA023 and other humanized TL1A antibodies were diluted to 800 nM using the medium, followed by 3-fold serial dilution to generate 9 concentration gradients, and 50 µL/well was added to the aforementioned 3599 cell plate. Thus, the final concentration of TL1A-his was 200 ng/mL, the final concentration of CHX was 10 µg/mL, and the initial final concentration of TL1A antibody was 200 nM. The cell plate was gently tapped to mix and then placed in a 37°C incubator. After 6 hours, the cell plate was removed and centrifuged at 500 g for 5 minutes. The supernatant was discarded, and the cells were washed twice with 1% BSA-PBS. Staining was performed using an Annexin V-FITC/PI Apoptosis Kit (purchased from Yeasen Biotechnology (Shanghai) Co., Ltd., Cat. No.: 40302ES60). The cells were resuspended in 1x Binding buffer at 100 µL/well. Then, 5 µL of Annexin V-FITC and 10 µL of PI Staining Solution were added to each well. After incubation at room temperature in the dark for 10 to 15 minutes, the mixture was homogenized and analyzed using a flow cytometer. Data analysis was performed with GraphPad Prism 9 to generate graphs and calculate the EC₅₀. The results are shown in Figure 6. The experimental results indicate that the candidate antibodies 311V25-7, 311V31-7, 311V34-7, and the positive control antibody PRA023 all inhibited TL1A-induced apoptosis in TF-1 cells, with the candidate antibodies exhibiting superior inhibitory activity compared to the positive control antibody PRA023.

### Example 8: Inhibition of TL1A-Stimulated IFN-γ Secretion by Anti-TL1A Antibodies

TL1A exerts its stimulatory effect on IFN-γ secretion by binding to DR3. This example measures the activity of anti-TL1A antibodies in inhibiting TL1A-induced IFN-γ secretion in PBMCs.

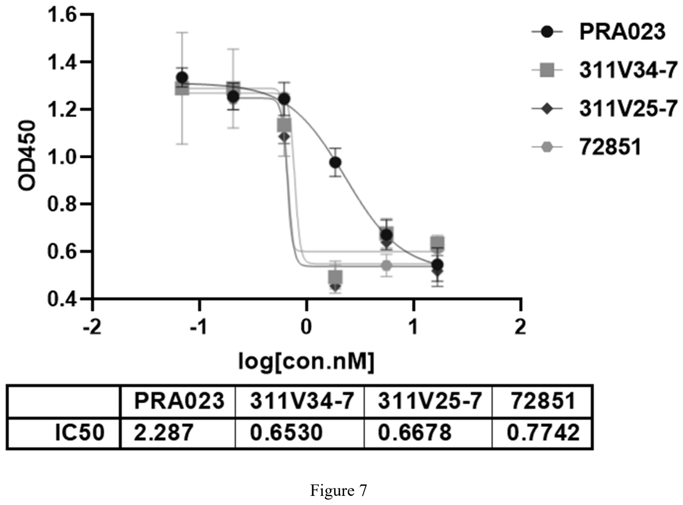

PBMCs were diluted in RPMI 1640 + 10% FBS medium, and the cell density was adjusted to 3×10⁶/mL. A 100 µL aliquot of the cell suspension was added to each well. TL1A-his was diluted to 400 ng/mL with medium, and antibodies were diluted to 200 nM and subjected to 3-fold gradient dilution. TL1A-his and antibodies were mixed at a volume ratio of 1:1, incubated at room temperature for 30 minutes, and then 50 µL of the mixture was added to each well of the PBMC plate. Recombinant IL12 protein was diluted to 4 ng/mL with medium, and 25 µL was added per well. Recombinant IL18 protein was diluted to 40 ng/mL with medium, and 25 µL was added per well to the aforementioned cell plate. The plate was incubated at 37°C with 5% CO₂ for 48 hours, after which the cell supernatant was collected, and the expression level of human IFN-γ in the supernatant was detected. The results are shown in Figure 7. The experimental results indicate that candidate antibodies 72851, 311V25-7, 311V34-7, and the positive control antibody PRA023 all inhibited IFN-γ secretion in PBMCs, with the candidate antibodies exhibiting superior inhibitory activity compared to the positive control PRA023.

### Example 9: Inhibition of TL1A-Stimulated TNF-α Secretion by Anti-TL1A Antibodies

As a Th1-polarizing factor, TL1A possesses the activity of stimulating Th1 cells to secrete cytokines such as IFN-γ and TNF-α, and thus may play an important role in the occurrence and development of inflammatory bowel disease (IBD). This example measures the activity of anti-TL1A antibodies in inhibiting TL1A-induced TNF-α secretion in PBMCs.

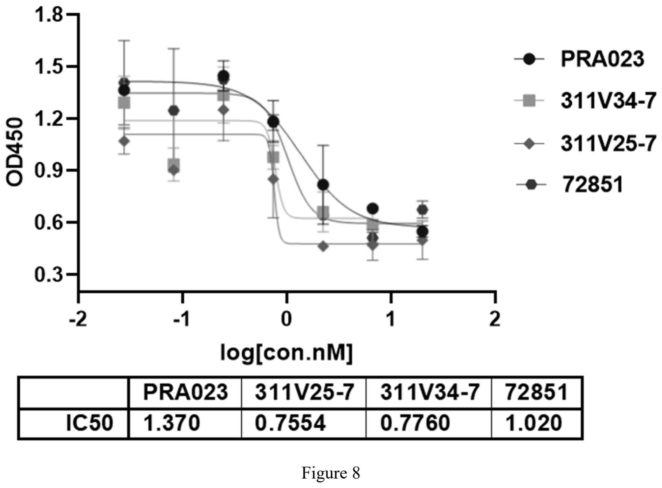

PBMCs were diluted in RPMI 1640 + 10% FBS medium, and the cell density was adjusted to 3×10⁶mL. A 100 µL aliquot of the cell suspension was added to each well. TL1A-his was diluted to 400 ng/mL with medium, and antibodies were diluted to 200 nM and 80 nM, followed by three-fold serial dilution. TL1A-his and antibodies were mixed at a volume ratio of 1:1, incubated at room temperature for 30 minutes, and then 50 µL of the mixture was added to each well of the aforementioned PBMC cell plate. Recombinant IL23 protein was diluted to 400 ng/mL with medium, and 25 µL was added per well. Recombinant IL2 protein was diluted to 80 U/mL with medium, and 25 µL was added per well to the aforementioned cell plate. The plate was incubated at 37°C with 5% CO₂ for 72 hours, after which the cell supernatant was collected, and the expression level of human TNF-α in the supernatant was detected. The results are shown in Figure 8. The experimental results indicate that candidate antibodies 72851, 311V25-7, 311V34-7, and the positive control antibody PRA023 all inhibited TNF-α secretion in PBMCs, with the candidate antibodies exhibiting superior inhibitory activity compared to the positive control PRA023.

### Example 10 Pharmacodynamic Study of Anti-TL1A Antibodies in a DSS-Induced Chronic Inflammatory Bowel Disease Model in Rats

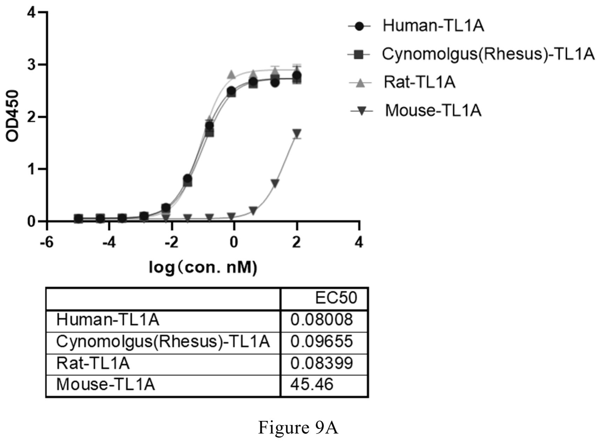

DSS was dissolved in the drinking water of rats. The high negative charge carried by its sulfate groups disrupts the integrity of the intestinal mucosal barrier, inducing an inflammatory response. The animals exhibited significant weight loss, loose stools, bloody stools, and granulocyte infiltration, demonstrating clinical symptoms and pathological features highly similar to those of human ulcerative colitis. The candidate anti-TL1A antibody molecule 72851 exhibits comparable binding activity to TL1A homologous proteins of human (human-TL1A), cynomolgus monkey (which shares the same sequence as rhesus monkey, Cynomolgus(Rhesus)-TL1A), and rat (Rat-TL1A) (ELISA experimental results are shown in Figure 9A). Therefore, a DSS-induced chronic inflammatory bowel disease model in rats was employed for in vivo pharmacodynamic evaluation.

The experiment was divided into three groups: a blank control group, a model group, and a 72851 treatment group. The blank control group received normal purified drinking water, while the model group and the 72851 treatment group were provided with 5% DSS-containing drinking water for 5 days per week and purified drinking water for 2 days per week, with each cycle lasting 7 days. The experiment was conducted over a total of 4 cycles. 72851 was administered at a dose of 5 mg/kg once every two days. At the end of the experiment, animals were anesthetized for blood collection and serum isolation. Following euthanasia, the entire intestinal tract from the cecum to the distal rectum was dissected and measured for length. The spleen was also collected and weighed. Serum levels of IL-6 and IL-1β were measured using enzyme-linked immunosorbent assay (ELISA).

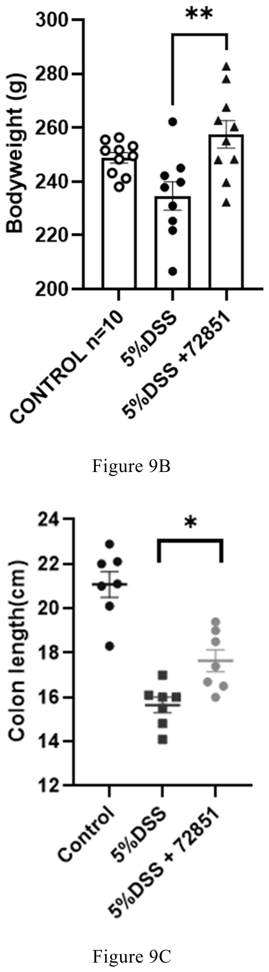

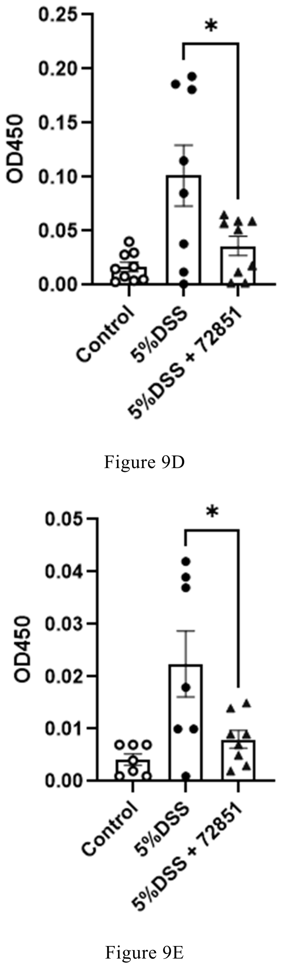

Experimental results demonstrated that, compared to the model group (5% DSS), candidate antibody 72851 in the 72851 treatment group (5% DSS + 72851) showed effective inhibition of weight loss (as shown in Figure 9B) and colon shortening (as shown in Figure 9C) in rats, as well as significant suppression of the increase in inflammatory cytokines IL-6 and IL-1β (as shown in Figures 9D and 9E).

### Example 11 Pharmacodynamic Study of Anti-TL1A Antibody in a TNBS-Induced Acute Inflammatory Bowel Disease Model in Rats

TNBS is a small molecule hapten that, upon rectal perfusion treatment, can bind to host proteins to induce an immune response, causing transmural necrosis and extensive inflammatory cell infiltration in the animal colon, accompanied by weight loss and intestinal obstruction. It is a commonly used modeling agent for simulating clinical Crohn's disease-like acute enteritis in animal models. We validated the efficacy of the anti-TL1A antibody 72851 in treating acute enteritis in rats using this model and compared it with RVT-3101 from Roche.

The experiment was divided into four groups: a blank control group, a model group, an RVT-3101 treatment group, and a 72851 treatment group. Before the experiment, animals were fasted for 18 hours. RVT-3101 and 72851 were administered at a dose of 5 mg/kg prior to modeling. Subsequently, animals in the model group, RVT-3101 treatment group, and 72851 treatment group were anesthetized, and 400 µL of a 1.5% TNBS ethanol solution (50% ethanol) was perfused into the colon via a plastic catheter through the anus. Rats were maintained in an inverted position for 3 minutes to prevent leakage of the perfusate. Animal conditions were monitored during anesthesia, and after awakening, animals were returned to their cages with free access to food and water. RVT-3101 and 72851 were administered via intraperitoneal injection once every two days; the model group received PBS injections every two days, with a administration volume of 5 mL/kg for all groups. On day 7 of the experiment, animals were anesthetized, blood was collected, and euthanasia was performed. The entire intestinal tract from the cecum to the distal rectum was excised for length measurement, and the spleen was collected and weighed.

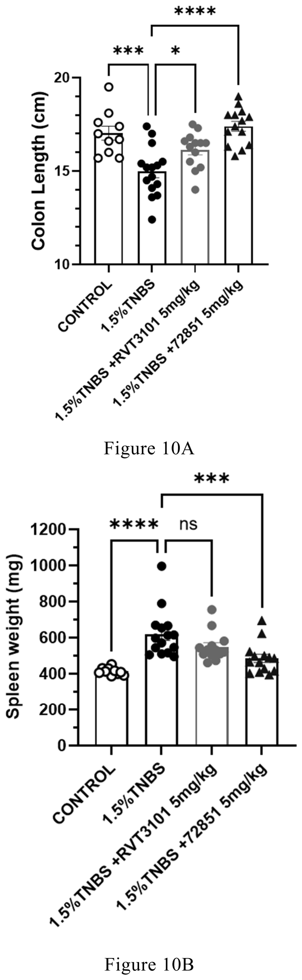

Experimental results demonstrated that compared to the model group (1.5% TNBS), candidate antibody 72851 in the 72851 treatment group (1.5% TNBS+72581) effectively inhibited colon shortening in rats (as shown in Figure 10A) and reduced the increase in spleen weight (as shown in Figure 10B), with efficacy superior to the positive control RVT-3101 treatment group (1.5% TNBS+RVT3101).

The foregoing embodiments are provided to illustrate the embodiments disclosed in the present invention and should not be construed as limitations to the present invention. Furthermore, various modifications and variations of the methods in the present invention, which are obvious to those skilled in the art, are intended to be within the scope and spirit of the present invention. Although the present invention has been described in connection with specific preferred embodiments, it should be understood that the invention is not limited to these specific embodiments. In fact, any modifications that are obvious to those skilled in the art for obtaining the invention, as described above, shall fall within the scope of the present invention.

## Claims

1. An anti-TL1A antibody or antigen-binding fragment thereof, wherein the anti-TL1A antibody or antigen-binding fragment thereof comprises a heavy chain variable region and a light chain variable region, and the anti-TL1A antibody or antigen-binding fragment thereof has one or more of the following technical features:
<1> the heavy chain variable region comprises an HCDR1 having an amino acid sequence as shown in SEQ ID NO: 10 or 18;
<2> the heavy chain variable region comprises an HCDR2 having an amino acid sequence as shown in SEQ ID NO: 11, 19, 39, 40, or 41;
<3> the heavy chain variable region comprises an HCDR3 having an amino acid sequence as shown in SEQ ID NO: 12, 20, or 42;
<4> the light chain variable region comprises an LCDR1 having an amino acid sequence as shown in SEQ ID NO: 13 or 21;
<5> the light chain variable region comprises an LCDR2 having an amino acid sequence as shown in SEQ ID NO: 14 or 22;
<6> the light chain variable region comprises an LCDR3 having an amino acid sequence as shown in SEQ ID NO: 15 or 23.

2. The anti-TL1A antibody or antigen-binding fragment thereof according to claim 1, wherein the anti-TL1A antibody or antigen-binding fragment thereof comprises:
<1> a heavy chain variable region comprising an HCDR1 having an amino acid sequence as shown in SEQ ID NO: 10;
<2> a heavy chain variable region comprising an HCDR2 having an amino acid sequence as shown in SEQ ID NO: 11;
<3> a heavy chain variable region comprising an HCDR3 having an amino acid sequence as shown in SEQ ID NO: 12;
<4> a light chain variable region comprising an LCDR1 having an amino acid sequence as shown in SEQ ID NO: 13;
<5> a light chain variable region comprising an LCDR2 having an amino acid sequence as shown in SEQ ID NO: 14;
<6> a light chain variable region comprising an LCDR3 having an amino acid sequence as shown in SEQ ID NO: 15;
and/or, the anti-TL1A antibody comprises:
<1> a heavy chain variable region comprising an HCDR1 having an amino acid sequence as shown in SEQ ID NO: 18;
<2> a heavy chain variable region comprising an HCDR2 having an amino acid sequence as shown in any one of SEQ ID NO: 19, 39, 40, and 41;
<3> a heavy chain variable region comprising an HCDR3 having an amino acid sequence as shown in SEQ ID NO: 20 or 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

3. The anti-TL1A antibody or antigen-binding fragment thereof according to claim 1 or 2, wherein the anti-TL1A antibody or antigen-binding fragment thereof further comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 19;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 39;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 40;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 41;
<3> a heavy chain variable region comprising an HCDR3 having an amino acid sequence as shown in SEQ ID NO: 20;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 39;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 40;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23;
and/or, the anti-TL1A antibody or antigen-binding fragment thereof comprises:
<1> a heavy chain variable region comprising an HCDR1 having an amino acid sequence as shown in SEQ ID NO: 18;
<2> a heavy chain variable region comprising an HCDR2 having an amino acid sequence as shown in SEQ ID NO: 41;
<3> a heavy chain variable region comprising an HCDR3 having an amino acid sequence as shown in SEQ ID NO: 42;
<4> a light chain variable region comprising an LCDR1 having an amino acid sequence as shown in SEQ ID NO: 21;
<5> a light chain variable region comprising an LCDR2 having an amino acid sequence as shown in SEQ ID NO: 22;
<6> a light chain variable region comprising an LCDR3 having an amino acid sequence as shown in SEQ ID NO: 23.

4. The anti-TL1A antibody or antigen-binding fragment thereof according to claim 1, wherein the heavy chain variable region of the anti-TL1A antibody or antigen-binding fragment thereof has an amino acid sequence as shown in any one of SEQ ID NO: 3, SEQ ID NO: 16, SEQ ID NO: 7, SEQ ID NO: 24, and SEQ ID NO: 26-31, and/or the light chain variable region of the anti-TL1A antibody or antigen-binding fragment thereof has an amino acid sequence as shown in SEQ ID NO: 5, SEQ ID NO: 17, SEQ ID NO: 9, or SEQ ID NO: 25.

5. The anti-TL1A antibody or antigen-binding fragment thereof according to claim 1, further comprising any one of the following features:
1) the heavy chain variable region of the anti-TL1A antibody or antigen-binding fragment thereof has an amino acid sequence as shown in SEQ ID NO: 3, and the light chain variable region has an amino acid sequence as shown in SEQ ID NO: 5;
2) the heavy chain variable region of the anti-TL1A antibody or antigen-binding fragment thereof has an amino acid sequence as shown in SEQ ID NO: 16, and the light chain variable region has an amino acid sequence as shown in SEQ ID NO: 17;
3) the heavy chain variable region of the anti-TL1A antibody or antigen-binding fragment thereof has an amino acid sequence as shown in SEQ ID NO: 7, and the light chain variable region has an amino acid sequence as shown in SEQ ID NO: 9;
4) the heavy chain variable region of the anti-TL1A antibody or antigen-binding fragment thereof has an amino acid sequence as shown in SEQ ID NO: 24, and the light chain variable region has an amino acid sequence as shown in SEQ ID NO: 25;
5) the heavy chain variable region of the anti-TL1A antibody or antigen-binding fragment thereof has an amino acid sequence as shown in SEQ ID NO: 26, and the light chain variable region has an amino acid sequence as shown in SEQ ID NO: 25;
6) the heavy chain variable region of the anti-TL1A antibody or antigen-binding fragment thereof has an amino acid sequence as shown in SEQ ID NO: 27, and the light chain variable region has an amino acid sequence as shown in SEQ ID NO: 25;
7) the heavy chain variable region of the anti-TL1A antibody or antigen-binding fragment thereof has an amino acid sequence as shown in SEQ ID NO: 28, and the light chain variable region has an amino acid sequence as shown in SEQ ID NO: 25;
8) the heavy chain variable region of the anti-TL1A antibody or antigen-binding fragment thereof has an amino acid sequence as shown in SEQ ID NO: 29, and the light chain variable region has an amino acid sequence as shown in SEQ ID NO: 25;
9) the heavy chain variable region of the anti-TL1A antibody or antigen-binding fragment thereof has an amino acid sequence as shown in SEQ ID NO: 30, and the light chain variable region has an amino acid sequence as shown in SEQ ID NO: 25;
10) the heavy chain variable region of the anti-TL1A antibody or antigen-binding fragment thereof has an amino acid sequence as shown in SEQ ID NO: 31, and the light chain variable region has an amino acid sequence as shown in SEQ ID NO: 25.

6. The anti-TL1A antibody or antigen-binding fragment thereof according to claim 1, wherein the anti-TL1A antibody or antigen-binding fragment thereof is selected from the group consisting of a full-length antibody, a single-chain antibody, or an antibody fragment; preferably, the heavy chain constant region of the full-length antibody is an IgG1 constant region, and/or the light chain constant region is a kappa chain constant region.

7. The anti-TL1A antibody or antigen-binding fragment thereof according to claim 1, wherein the heavy chain constant region of the anti-TL1A antibody or antigen-binding fragment thereof has an amino acid sequence as shown in SEQ ID NO: 33; and/or the light chain constant region of the anti-TL1A antibody or antigen-binding fragment thereof has an amino acid sequence as shown in SEQ ID NO: 34.

8. An isolated polynucleotide, wherein the polynucleotide encodes the heavy chain variable region and/or light chain variable region or full-length amino acid sequence of the anti-TL1A antibody or antigen-binding fragment thereof according to any one of claims 1-7.

9. The polynucleotide according to claim 8, wherein the polynucleotide encoding the heavy chain variable region of the anti-TL1A antibody or antigen-binding fragment thereof has a sequence as shown in SEQ ID NO: 2, SEQ ID NO: 6, SEQ ID NO: 35, SEQ ID NO: 37, SEQ ID NO: 43 to 48; and/or the polynucleotide encoding the light chain variable region of the anti-TL1A antibody or antigen-binding fragment thereof has a sequence as shown in SEQ ID NO: 4, SEQ ID NO: 8, SEQ ID NO: 36, SEQ ID NO: 38.

10. A nucleic acid construct, comprising the isolated polynucleotide according to claim 8 or 9.

11. A cell, wherein the cell contains the construct according to claim 10 or has an exogenous polynucleotide according to claim 8 or 9 integrated into its genome.

12. A method for preparing the anti-TL1A antibody or antigen-binding fragment thereof according to any one of claims 1 to 7, comprising the following steps: culturing the cell according to claim 11 under conditions suitable for expressing the TL1A antibody or antigen-binding fragment thereof, thereby expressing the anti-TL1A antibody or antigen-binding fragment thereof.

13. Use of the anti-TL1A antibody or antigen-binding fragment thereof according to any one of claims 1 to 7 in the preparation of a medicament for treating a disease or in the preparation of a medicament for diagnosing a disease; preferably, the disease is selected from one or more of inflammatory bowel disease, gastrointestinal diseases associated with cystic fibrosis, colitis, irritable bowel syndrome, eosinophilic esophagitis, atopic dermatitis, eczema, scleroderma, arthritis or rheumatoid arthritis.

14. The use according to claim 13, wherein the inflammatory bowel disease is Crohn's disease or ulcerative colitis.

15. A pharmaceutical composition, wherein the pharmaceutical composition comprises the anti-TL1A antibody or antigen-binding fragment thereof according to any one of claims 1 to 7.
